# EUROPEAN PATENT SPECIFICATION

(11) **EP 2 473 623 B1**
(45) Date of publication and mention of the grant of the patent: **08.07.2015**
(21) Application number: 10747860.4
(22) Date of filing: 31.08.2010
(51) Int. Cl.: C12Q 1/68, C12N 15/10

(54) **PURIFICATION PROCESS OF NASCENT DNA**
VERFAHREN ZUR AUFREINIGUNG VON NASZIERENDER DNA
PROCÉDÉ DE PURIFICATION D'ADN NAISSANT

(30) Priority: 31.08.2009 US 238315 P
(43) Date of publication of application: 11.07.2012
(73) Proprietor: Centre National de la Recherche Scientifique (CNRS), 75794 Paris Cedex 16 (FR)
(72) Inventor: MECHALI, Marcel, 34980 Montferrier sur Lez (FR); COULOMBE, Philippe, 34090 Montpellier (FR); CAYROU, Christelle, 34000 Montpellier (FR); RIVALS, Eric, 34070 Montpellier (FR)
(74) Representative: Monni, Richard
(86) International application number: PCT/EP2010/062736
(87) International publication number: WO 2011/023827

(56) References cited:
- WO-A1-2009/099326
- WO-A2-2004/015063
- PRIOLEAU MARIE-NOELLE ET AL: "Replication of the chicken beta-globin locus: Early-firing origins at the 5' HS4 insulator and the rho- and betaA-globin genes show opposite epigenetic modifications.", MOLECULAR AND CELLULAR BIOLOGY, vol. 23, no. 10, May 2003 (2003-05), pages 3536-3549, XP002603564, ISSN: 0270-7306
- CADORET JEAN-CHARLES ET AL: "Genome-wide studies highlight indirect links between human replication origins and gene regulation", PROCEEDINGS OF THE NATIONAL ACADEMY OF SCIENCES OF THE UNITED STATES OF AMERICA, vol. 105, no. 41, October 2008 (2008-10), pages 15837-15842, XP002603565, ISSN: 0027-8424 cited in the application
- GOMEZ MARIA ET AL: "Overreplication of short DNA regions during S phase in human cells", GENES & DEVELOPMENT, vol. 22, no. 3, February 2008 (2008-02), pages 375-385, XP002603566, ISSN: 0890-9369
- SEQUEIRA-MENDES JOANA ET AL: "Transcription Initiation Activity Sets Replication Origin Efficiency in Mammalian Cells", PLOS GENETICS, vol. 5, no. 4, April 2009 (2009-04), XP002603567, cited in the application
- DAS-BRADOO SAPNA ET AL: "Replication Initiation Point Mapping: Approach and Implications", 23 January 2009 (2009-01-23), METHODS IN MOLECULAR BIOLOGY HUMANA PRESS INC, 999 RIVERVIEW DR, STE 208, TOTOWA, NJ 07512-1165 USA SERIES : METHODS IN MOLECULAR BIOLOGY (ISSN 1064-3745(PRINT)), XP008127649, ISSN: 978-1-60327-814-0(H) vol. 521, pages 105-120, page 14
- DATABASE Geneseq [Online] 1 November 2007 (2007-11-01), "VIC-labeled probe for marker 30026 detection.", XP002616997, retrieved from EBI accession no. GSN:AJF62338 Database accession no. AJF62338
- DATABASE Geneseq [Online] 20 February 2002 (2002-02-20), "Oligonucleotide SEQ ID NO 5406 for detecting SNP TSC0001818.", XP002616998, retrieved from EBI accession no. GSN:ABC05415 Database accession no. ABC05415
- DATABASE Geneseq [Online] 12 March 2002 (2002-03-12), "PCR primer (dCT)6 used in method of identifying microorganisms.", XP002616999, retrieved from EBI accession no. GSN:AAS18633 Database accession no. AAS18633
- DATABASE Geneseq [Online] 1 November 2007 (2007-11-01), "VIC-labeled probe for marker 30026 detection.", retrieved from EBI accession no. GSN:AJF62338 Database accession no. AJF62338
- LAURIE C C: "VIC-labeled probe for marker 30026 detection", GENESEQ,, 15 February 2007 (2007-02-15), XP002616997, [retrieved on 2007-11-01]
- PARSONS B J ET AL: "CONTRASTING GENETIC DIVERSITY RELATIONSHIPS ARE REVEALED IN RICE (ORYZA SATIVA L.) USING DIFFERENT MARKER TYPES", MOLECULAR BREEDING: NEW STRATEGIES IN PLANT IMPROVEMENT, KLUWER ACADEMIC PUBLISHERS, NL, vol. 3, no. 2, 1 January 1997 (1997-01-01) , pages 115-125, XP001149290, ISSN: 1380-3743, DOI: 10.1023/A:1009635721319
- J. Ji ET AL: "In Vitro Expansion of GGC:GCC Repeats: Identification of the Preferred Strand of Expansion", Nucleic Acids Research, vol. 24, no. 14, 1 July 1996 (1996-07-01), pages 2835-2840, XP55109755, ISSN: 0305-1048, DOI: 10.1093/nar/24.14.2835

## Description

The present invention relates to a purification process of nascent DNA.

In metazoans, thousands of chromosomal sites are activated at each cell cycle to initiate DNA synthesis and permit total duplication of the genome. They all should be activated only once to avoid any amplification and maintain genome integrity. How these sites are defined remains elusive despite considerable efforts trying to unravel a possible replication origin code.
In *Saccharomyces cerevisiae*, DNA replication origins are specifically identified by specific DNA elements, called Autonomous Replication Sequence elements (ARS), which have a common AT-rich 11 bp specific consensus. However, sequence specificity identifies but not determines origin selection.
In multicellular organisms, it was more difficult to identify common features of DNA replication origins. No consensus sequence element has been found, which can have predictive value, although specific sites are recognized as DNA replication origins in chromosomes of somatic cells.
The identification of the sequence of DNA replication origin offers new perspectives in the comprehension of pathologies involving miss regulation of DNA replication, and new perspective in the cellular therapy, by using "humanized" vectors.

International application WO 98/27200 discloses a putative consensus sequence of human and mammalian replication origin. However, the consensus sequences disclosed in WO 98/27200 appears to be not representative of all the replications origins normally used in multicellular eukaryotic cells.
Prior art also discloses methods for purifying nascent DNA for mapping of DNA replication origins in multicellular eukaryotic cells [Prioleau et al. 2003, Molecular and Cellular Biology, 23(10), pages 3536-3549*;* Cadoret et al. 2008, P.N.A.S., 105(41), pages 15837-15842*;* Gomez et al. 2008, Genes & Development, 22(3), pages 375-385*;* Sequeira-Mendes, 2009, PloS Genetics, 5(4*)*].
Prioleau et al. 2003, Molecular and Cellular Biology, 23(10), pages 3536-3549 disclose methods for purification of nascent strands. The method included sucrose gradients, heat denaturation and exonuclease digestion. The heat denaturation step eliminates proteins associated with nucleic acid molecules.

So, there is a need to provide a new consensus sequence representing all the DNA replication origins of a multicellular eukaryotic cell.
There is also a need to provide a new method for determining the DNA replication origins of a multicellular eukaryotic cell.

One aim of the invention is to provide a method for purifying nascent DNA in a large amount and with a very high purity.

One aim of the invention is to provide a method for identifying eukaryotic replication origin.
Another aim of the invention is to provide the sequence of said eukaryotic replication origin.
Another aim is the use of nascent DNA produced by said replication origin for providing a method of diagnosis.

The disclosure relates to the use of purified nascent DNA (hybrid RNA-DNA) for the implementation of a process allowing the mapping and the numbering of the active DNA replication origins of multi cellular eukaryotic cells, and the characterisation of the sequence of said replication origins,
said process comprising
- a step of extracting a mixture of nucleic acid molecules, said mixture of nucleic acid molecules comprising DNA and hybrid RNA-DNA, from multi cellular eukaryotic cells
- a step of enrichment of hydrid RNA-DNA from said mixture by eliminating proteins associated with said nucleic acid molecules, and
- at least two step of elimination of DNA from the mixture to recover purified nascent DNA.

The initiation of new DNA strands at origins of replication in multicellular eukaryotic cells requires de novo synthesis of RNA primers by primase and subsequent elongation from RNA primers by DNA polymerase alpha. These nascent DNA are thus hybrid molecules consisting of a short molecule of RNA fused in its 3' end to a DNA molecule. The inventors have unexpectedly discovered that eliminating proteins associated with DNA (histones for instance), allow a large increase in the purifying efficiency of nascent DNA.
Also, the inventors have demonstrated that a double cycle of phosphorylation/digestion of lambda exonuclease also drastically enhances the purity of isolated nascent DNA.
The nascent DNA are purified, which means that said nascent DNA are substantially pure: after one step of exonuclease, contaminant DNA represent about 25% of the purified DNA.

According to the disclosure, at least two exonuclease treatments allows to eliminate contaminant DNA (after 2 steps: about 5% of DNA is present in the mixture, after 3 steps less than 2% of contaminant DNA is present in the mixture).
In one advantageous embodiment, the disclosure relates to the use as defined above, wherein said nascent DNA are produced by the active replication origins.

In one advantageous embodiment, the disclosure relates to the use of purified nascent DNA for mapping and numbering the active DNA replication origins as defined above, wherein said process is carried out by using multicellular organism totipotent cells. In totipotent cells, such as ES cells, all the DNA replication origins are active, to allow a rapid duplication of DNA

In one advantageous embodiment, the i disclosure relates to the use of purified nascent DNA for mapping and numbering the active DNA replication origins as defined above, wherein process is carried out by using multicellular organism differentiated cells.
In differentiated cells, not all the DNA replication origins are active.

In one advantageous embodiment, the disclosure relates to the use of purified nascent DNA for the characterisation of the sequence the active DNA replication origins as defined above, wherein said sequence consists of
- the nucleic acid sequences selected from the group comprising the following sequences:
   5'- (N₇)ₐ(N₈)_{b}(GN₁N₂)_{c}(N₇)_{d}(N8)ₑ-3' (SEQ ID NO: 1)
      wherein N₁ is a G or a A and N₂ is a pyridine or a A
   5'- (N₇)ₐ(Nₛ)_{b}(N₃GN₄)_{c}(N₇)_{d}(Nₛ)ₑ-3' (SEQ ID NO: 2)
      wherein N₃ is a Tor a G base and N₄ is a G or a C, and
   5'- (N₇)ₐ(N₈)_{b}(N₅N₆G)_{c}(N₈)_{d}(N₉)ₑ-3' (SEQ ID NO: 3)
      wherein Nₛ is different from N₆, N₅ is a G or a C and N₆ is a Tor a A
   wherein c vary from 3 to 20
   wherein N₇ and N₈ represent any nucleotide,
   wherein a and e independently from each other can be equal to 0, 1 2 or 3, or vary from about 15 to 30, and
   wherein band d independently from each other can be equal to 0, 1 2 or 3 or vary from about 10 to 300,
   N₈ being such that if b vary from 10 to 300, (N₈)_{b} represents a nucleic acid chain which is such that
   ▪ it contains from about 50% to about 100% of A,
   ▪ it contains from about 50% to about 100% of T,
   ▪ it contains from 0% to about 10% of G, and
   ▪ it contains from 0% to about 12 % of C,
   N₉ being such that if d vary from 10 to 300, (N₉)_{d} represents a nucleic acid chain which is such that
   ▪ it contains from about 50% to about 100% of A,
   ▪ it contains from about 50% to about 100% of T,
   ▪ it contains from 0% to about 10% of G, and
   ▪ it constrains from 0% to about 12% of C.
- or any fragment of the above sequence consisting of at least 9 nucleotides.

In the invention pyridine means T or C, or U for RNA.

In one other embodiment, the disclosure relates to the use of purified nascent DNA as defined above, wherein said nucleic acid sequence being such that
▪ it contains from about 33% to about 66% of G,
▪ it contains from about 27% to about 33% of C,
▪ it contains from about 0% to about 12% of A,
▪ it contains from about 0% to about 15 % of T, and
▪ it has a minimal consensus sequence chosen among the following consensus sequences:
▪ 5'-N1N2G-3'
   wherein N1 is a Gor a A and N2 is a pyridine or a A
▪ S'-N3GN4-3'
   wherein N3 is a T or a G base and N4 is a G or a C, and
▪ 5'-GN5N6-3'
wherein Ns is different from N6, N5 is a G or a Cand N6 is a T or a A
said minimal consensus sequence being repeated from 3 to 20 times without interruption between said repeated minimal consensus sequence.

In one other embodiment, the disclosure relates to the use of purified nascent DNA .as defined above, wherein said nucleic acid sequence consists of the following sequence SEQ ID NO: 4:
5'-SHGCYGSYGGMGCYGSHGSTG-3',
   or any fragment of said nucleic acid sequence consisting of at least 9 nucleotides.

In the invention, the following nomenclature in nucleic acid sequence is used:
R represents A or G
Y represents C or T
M represents A or C
K represents G or T
S represents G or C
W represents A or T
B represents G, Tor C D represents G, A or T
H represents A, C or T
V represents G, C or A, and
N represents any nucleotide (A, T, G or C)

In one other embodiment, the disclosure relates to the use of purified nascent DNA as defined above, wherein said nucleic acid sequence consists of the following sequence SEQ ID NO: 5:
5' -CKGYKGCKGCDGCKGCDGYKG-3'
   or any fragment of said nucleic acid sequence consisting of at least 9 nucleotides. In one other embodiment, the disclosure relates to the use of purified nascent DNA as defined above, wherein said nucleic acid sequence consists of one of the following sequences
   GTCCCAGTCCCAG (SEQ ID NO: 6)
   TGCTGCTGCTGCT (SEQ ID NO: 7)
   TATATATATATAT (SEQ ID NO: 8)
   AGCAGCAGCAGCA (SEQ ID NO: 9)
   GTTGCTGCTGCTG (SEQ ID NO: 10)
   TCAGACATCTTAG (SEQ ID NO: 11)
   AGCAGCAGCAACA (SEQ NO: 12)
   CAGACATCTTAGG (SEQ ID NO: 13)
   AGACATCTTAGGC (SEQ ID NO: 14)
   CAGCAGCAGCAGC (SEQ ID NO: 15)
   TAACGTGTGGTGA (SEQ ID NO: 16)
   TGTTGCTGCTGCT (SEQ ID NO: 17)
   CAGCAGCAGCAAC (SEQ ID NO: 18)
   TGCTGCTGC (SEQ ID NO: 19)
   CAGCAGCAG (SEQ ID NO: 20)
   CTGCTGCTG (SEQ ID NO: 21)
   CTCTCTCTCTCT (SEQ ID NO: 22)
   TCTCTCTCTCTC (SEQ ID NO: 23)
   AGCTGGGGCGGCA (SEQ ID NO: 24)
   CAGCTGGGGCGGC (SEQ ID NO: 25)
   GCTGGGGCGGCAG (SEQ ID NO: 26)
   AGCAGCTGGACAC (SEQ ID NO: 27)
   CAGCAGCTGGACA (SEQ ID NO: 28)
   GCAGCAGCTGGAC (SEQ ID NO: 29)
   CAGCTGGACACAC (SEQ ID NO: 30)
   AGCAGACTGGGCG (SEQ ID NO: 31).

The disclosure also relates to an isolated nucleic acid sequence representing an multi cellular DNA replication origins, wherein said nucleic acid sequence consists of one of the following sequences
• the nucleic acid sequences selected from the group comprising the following sequences:
   5'- (N₇)ₐ(N₈)_{b}(GN₁N₂)_{c}(N₇)_{d}(N₈)ₑ-3' (SEQ ID NO: 1)
      wherein N₁ is a G or a A and N₂ is a pyridine or a A
   5'- (N₇)ₐ(N₈)_{b}(N₃GN₄)_{c}(N₇)_{d}(N₈)ₑ-3' (SEQ ID NO: 2)
      wherein N₃ is a Tor a G base and N₄ is a G or a C, and
   5'- (N₇)ₐ(N₈)_{b}(N₅N₆G)_{c}(N₈)_{d}(N₉)ₑ-3' (SEQ ID NO: 3)
      wherein N₅ is different from N₆, N₅ is a G or a C and N₆ is a Tor a A
   wherein c vary from 3 to 20
   wherein N₇ and N₈ represent any nucleotide,
   wherein a and e independently from each other can be equal to 0, 1, 2 or 3, or vary from about 15 to 30, and
   wherein band d independently from each other can be equal to 0, 1,2 or 3 or vary from about 10 to 300,
   N₈ being such that if b vary from 10 to 300, (N₈)_{b} represents a nucleic acid chain which is such that
▪ it contains from about 50% to about 100% of A,
▪ it contains from about 50% to about 100% of T,
▪ it contains from 0% to about 10% of G, and
▪ it contains from 0% to about 12% of C,
   N9 being such that if d vary from 10to 300, (N9)d represents a nucleic acid chain which is such that
▪ it contains from about 50% to about 100% of A,
▪ it contains from about 50% to about 100% of T,
▪ it contains from 0% to about 10% of G, and
▪ it contains from 0% to about 12% of C.
• or any fragment of the above sequence consisting of at least 9 nucleotides.

The invention relates to the use of an isolated nucleic acid sequence, as a multi cellular DNA replication origin wherein said nucleic acid sequence consists of
a) the nucleic acid sequence
   5'- (N₇)ₐ(N₈)_{b}(N₃GN₄)_{c}(N₇)_{d}(N₈)ₑ-3' (SEQ ID NO: 2)
      wherein N₃ is a T or a G base and N₄ is a G or a C,
      wherein c vary from 3 to 20
      wherein N₇ and N₈ represent any nucleotide,
      wherein a and e independently from each other can be equal to0, 1, 2 or 3, or vary from about 15 to 30, and
      wherein b can be equal to 0, 1, 2 or 3 wherein d can be equal to 0, 1, 2 or 3 or vary from about 10 to 300,
b) or any fragment of the above sequence consisting of at least 9 nucleotides, said nucleic acid sequence being such that
   - it contains from 33% to 66% of G,
   - it contains from 27% to 33% of C,
   - it contains from 0% to 12% of A,
   - it contains from 0% to 15% of T,
   - it has a minimal consensus sequence 5'-N3GN4-3' wherein N3 is a T or a G base and N4 is a G or a C
said minimal consensus sequence being repeated from 3 to 20 times without interruption between said repeated minimal consensus sequence.
Advantageously, the invention relates to the use of the isolated nucleic acid sequence according the above definition, wherein said nucleic acid sequence consists of one of the following sequences:
AGCTGGGGCGGCA (SEQ ID NO: 24)
CAGCTGGGGCGGC (SEQ ID NO: 25), and
GCTGGGGCGGCAG (SEQ ID NO: 26).
The above sequences that correspond to DNA eukaryotic origins are novel.

The disclosure relates to the isolated nucleic acid sequence according to claim 10, wherein said nucleic acid sequence being such that
▪ it contains from about 33% to about 66% of G,
▪ it contains from about 27% to about 33% of C,
▪ it contains from about 0% to about 12% of A,
▪ it contains from about 0% to about 15 % ofT, and
▪ it has a minimal consensus sequence chosen among the following consensus sequences:
▪ 5'-N₁N₂G-3'
   wherein N₁ is a G or a A and N₂ is a pyridine or a A
▪ 5'-N3GN₄-3'
   wherein N₃ is a T or a G base and N₄ is a G or a C, and
▪ 5'-GN₅N₆-3'
   wherein N₅ is different from N₆, Ns is a G or a C and N₆ is a Tor a A
said minimal consensus sequence being repeated from 3 to 20 times without interruption between said repeated minimal consensus sequence.

In one advantageous embodiment, the disclosure relates to the isolated nucleic acid sequence as defined above, wherein said nucleic acid sequence consists of the following sequence SEQ ID NO : 4:
5'-SHGCYGSYGGMGCYGSHGSTG-3'3
   or any fragment of said nucleic acid sequence consisting of at least 9 nucleotides.

In one advantageous embodiment, the disclosure relates to the isolated nucleic acid sequence as defined above, wherein said nucleic acid sequence consists of the following sequence SEQ ID NO: 5:
5'-CKGYKGCKGCDGCKGCDGYKG-3'
   or any fragment of said nucleic acid sequence consisting of at least 9 nucleotides.

In one advantageous embodiment, the disclosure relates to the isolated nucleic acid sequence as defined above, wherein said nucleic acid sequence consists of one of the following sequences
GTCCCAGTCCCAG (SEQ ID NO: 6)
TGCTGCTGCTGCT (SEQ ID NO: 7)
TATATATATATAT (SEQ ID NO: 8)
AGCAGCAGCAGCA (SEQ ID NO: 9)
GTTGCTGCTGCTG (SEQ ID NO: 10)
TCAGACATCTTAG (SEQ ID NO: 11)
AGCAGCAGCAACA (SEQ ID NO: 12)
CAGACATCTTAGG (SEQ ID NO: 13)
AGACATCTTAGGC (SEQ ID NO: 14)
CAGCAGCAGCAGC (SEQ ID NO: 15)
TAACGTGTGGTGA (SEQ ID NO: 16)
TGTTGCTGCTGCT (SEQ ID NO: 17)
CAGCAGCAGCAAC (SEQ ID NO: 18)
TGCTGCTGC (SEQ ID NO: 19)
CAGCAGCAG (SEQ ID NO: 20)
CTGCTGCTG (SEQ ID NO: 21)
CTCTCTCTCTCT (SEQ ID NO: 22)
TCTCTCTCTCTC (SEQ ID NO: 23)
AGCTGGGGCGGCA (SEQ ID NO: 24)
CAGCTGGGGCGGC (SEQ ID NO: 25)
GCTGGGGCGGCAG (SEQ ID NO: 26)
AGCAGCTGGACAC (SEQ ID NO: 27)
CAGCAGCTGGACA (SEQ ID NO: 28)
GCAGCAGCTGGAC (SEQ ID NO: 29)
CAGCTGGACACAC (SEQ ID NO: 30)
AGCAGACTGGGCG (SEQ ID NO: 31)

The disclosure also relates to a recombinant vector comprising at least one isolated nucleic acid sequence as defined above.
The above vector contains at least one origin of replication that replicates as the endogenous chromosomal DNA replication origins. Therefore, the vector is duplicated as an "endogenous chromosome". The Inventors have shown that this replication is effective (the above origins are active).

The invention also relates to a method, preferably in vitro, for controlling the replication of a nucleotidic sequence into a pluricellular eukaryotic cell, including mammal cells, comprising the insertion of, into said nucleotidic sequence, a nucleic acid sequence as defined above.

In one advantageous embodiment, the invention relates to the method as defined above, comprising a step of introducing said nucleotidic sequence into a pluricellular eukaryotic cell.

In one advantageous embodiment, the disclosure relates to the method as defined above for treating pathologies involving a deregulation of DNA replication, said method comprising the administration to an individual in a need thereof of a pharmaceutically effective amount of a nucleic acid sequence as defined above.

In one advantageous embodiment, the disclosure relates to the use of a nucleic acid sequence as defined above, for the preparation of a drug intended for the treatment of pathologies involving a deregulation of DNA replication.
In one advantageous embodiment, the invention relates to a nucleic acid sequence as defined above, for its use for the treatment of pathologies involving a deregulation of DNA replication.

The disclosure also relates to a pharmaceutical composition comprising, in particular as active substance, a nucleic acid sequence as defined above, in association with a pharmaceutically acceptable carrier.

The disclosure also relates to a map referencing all the DNA replication organisms of multicellular eukaryotic cells, said map being obtainable by the process as defined above.

The invention also relates to a map referencing all the DNA replication origins of multicellular eukaryotic totipotent cells, said map being obtainable by the process as defined above.

The invention also relates to a map referencing all the DNA replication origins activated in multicellular eukaryotic differenciated cells, said map being obtainable by the process as defined above.

The disclosure also relates to a method for the diagnostic, preferably in vitro or ex vivo, of pathologies involving a deregulation of DNA replication in an individual, or in a biological sample from an individual, said method comprising the steps:
- establishing the map referencing all DNA replication origins activated in multicellular eukaryotic differentiated cells of said individual, or of said biological sample from an individual,
- comparing the map obtained in the previous step with a reference map, said reference map corresponding to the map referencing all DNA replication origins activated in multicellular eukaryotic healthy differentiated cells of said individual or of said biological sample from an individual,
- concluding, from the previous comparison if said individual is afflicted by a pathology involving a deregulation of DNA replication

The disclosure also relates to a method for the diagnostic, preferably in vitro or ex vivo, of the genetic modification of a cell of an individual, preferably a pluripotent cell, said method comprising the steps:
- establishing the map referencing all DNA replication origins activated in a cell of an individual,
- comparing the map obtained in the previous step with a reference map, said reference map corresponding to the map referencing all DNA replication origins activated in an healthy cell of the same type than the cell used in the previous step,
- concluding, from the previous comparison said cell have a genetic modification (genetic variation of a cell during passages in vitro).

The invention also relates to a process for purifying nascent DNA, said process comprising
- a step of extracting a mixture of nucleic acid molecules, said mixture of nucleic acid molecules comprising DNA and hybrid RNA-DNA, from multi cellular eukaryotic cells
- a step of enrichment of hydrid RNA-DNA from said mixture by eliminating proteins associated with said nucleic acid molecules; and
- at least two step of elimination of DNA from the mixture to recover purified nascent DNA.

In one advantageous embodiment, the invention relates to the process as defined above, for purifying nascent DNA allowing the localisation and the numbering of the active DNA replication origins of multi cellular eukaryotic cells, said process comprising the steps:
- Harvesting and lysing dividing cells to obtain DNA,
- Digesting proteins anchored in DNA,
- Washing DNA in ethanol,
- Purifying nascent DNA in a single neutral 5 to 30% sucrose gradient,
- Collecting fractions corresponding to DNA from about 0.5-1 kb, about 1-1.5 kb, about 1.5-2 kb and about 2-3 kb,
- Phosphorylating extremity of DNA and digesting contaminant double strand DNA
- Repeating at least once the previous step
- Precipitating nascent DNA and purifying said nascent DNA (for instance with Cyscibe-GFX kit)
- Amplifying nascent DNA (for instance WGA-Sigma kit),
- Purifying amplicons, sais amplicons being the products of the amplification of said nacent DNA obtained in the previous step,
- Locating on whole genome nascent DNA (by DNA hybridization on WCA chip or by systematic DNA sequence of amplified DNA) to determine active replication origins, and numbering said active replication origins.

In one advantageous embodiment, the invention relates to the process as defined above, for purifying nascent DNA allowing the localisation and the numbering of all the DNA replication origins of multi cellular eukaryotic cells, said process being carried out in totipotent cells, wherein all the replication origins are actives.

### Figures

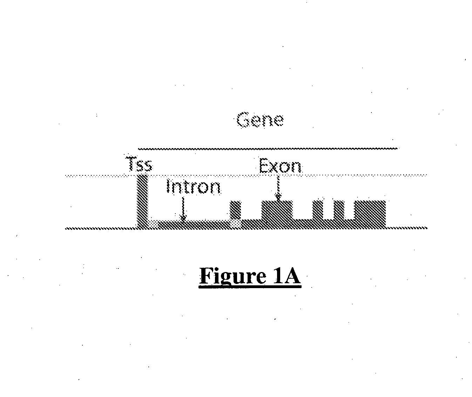

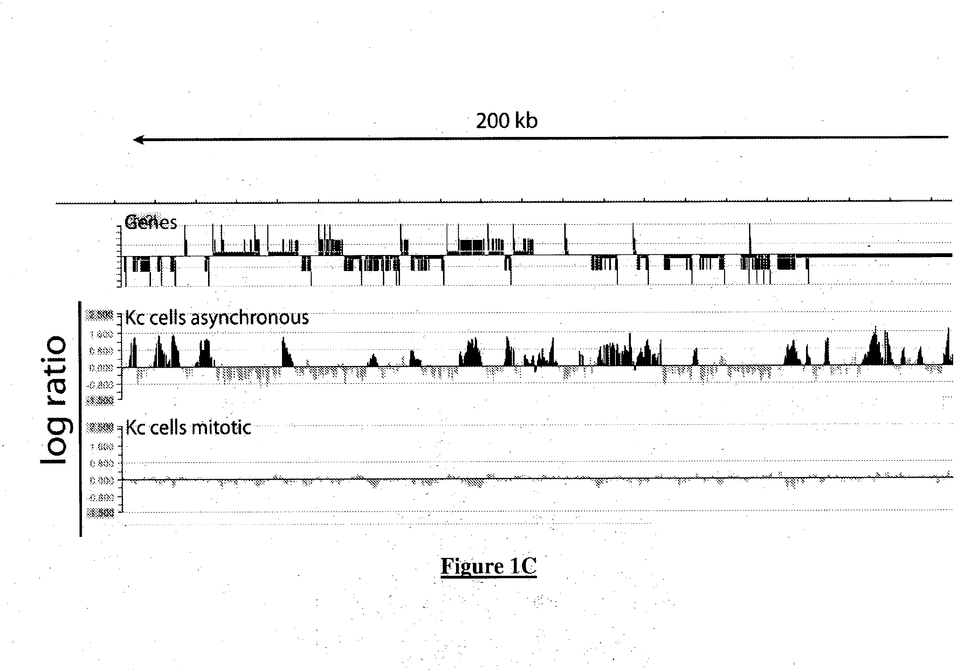

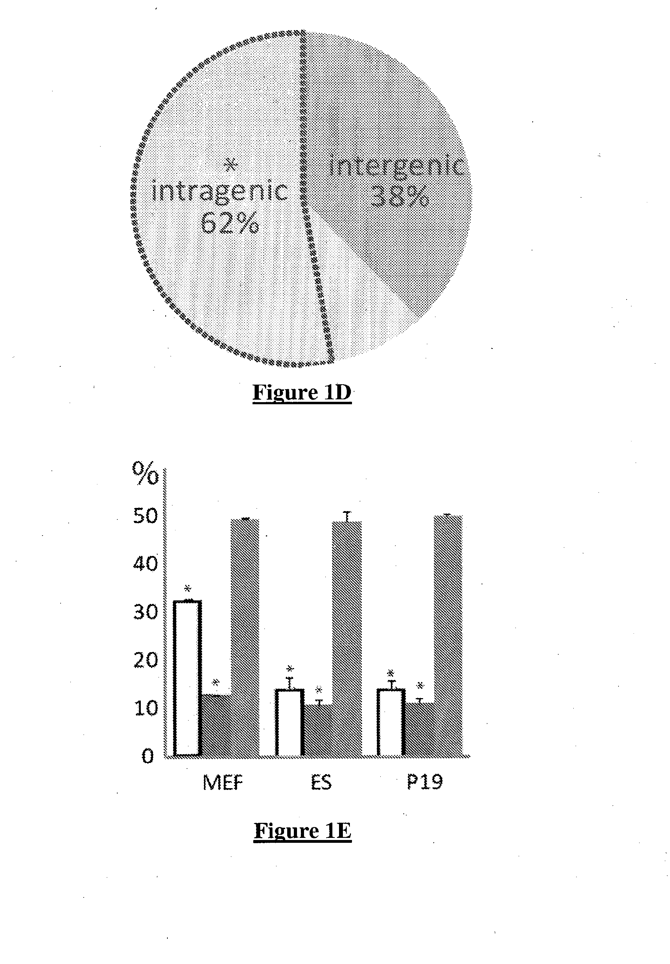

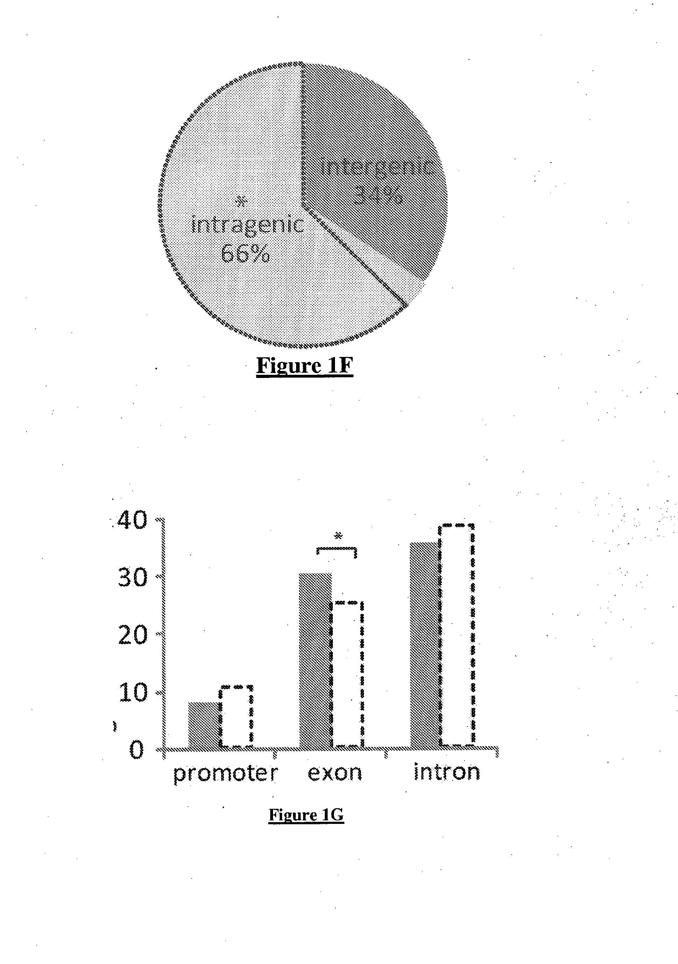

### Figures 1 A -I represent the association between genes and replication origins

Figure IA corresponds to a schematic representation of a gene, in which Tss (transcription initiation site), exon and intron are represented.

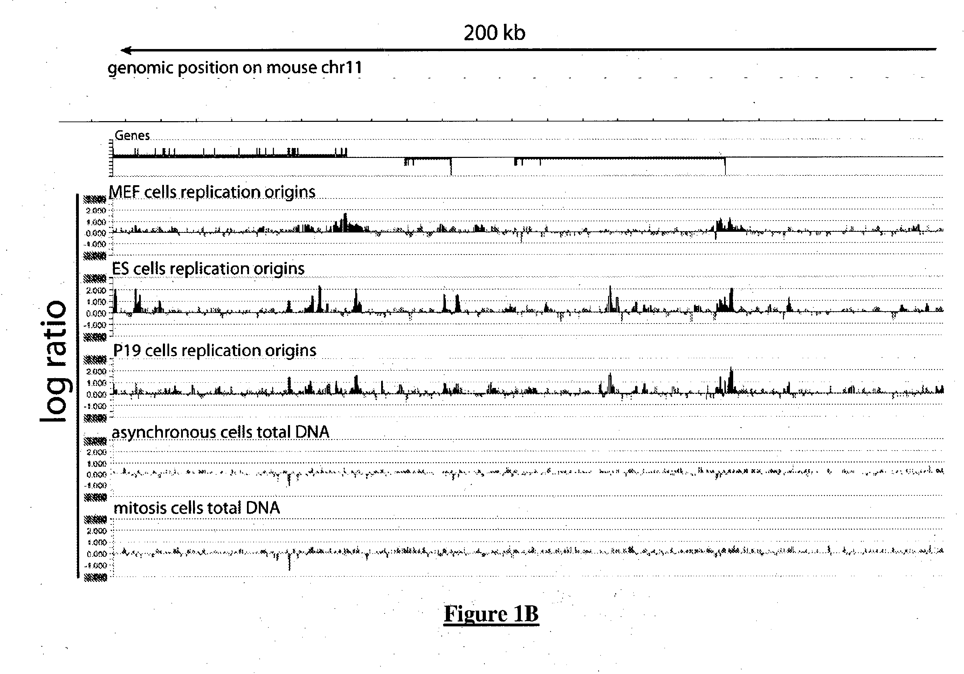

Figure 1B represents an example of the distribution of replication origins found on a 200 kb region of MEF cells and ES cells. Negative controls for the mouse cells are the P19 asynchronous cells or PI9 arrested in late mitosis by nocodazol.
Figure IC represents an example of the distribution of replication origins found on a 200 kb region of Kc cells. Negative control for Kc cells come from fragmented total DNA of mitotic cells and then treated by lambda exonuclease.
Figure ID represents a pie chart showing the percentage of origin sequences in genes sequences (light grey) and intergenic sequences (dark grey) in MEF cells. The value of gene association for randomized origins is indicated by the dashed pie (53%). Similar values were obtained for ES and P19 cells. (*:p<0.001)
Figure IE represents a graph showing the percentage of origin sequences in promotor sequences (white) and intronic sequences (light grey) and exonic sequences (dark grey) in MEF cells, ES cells and PI9 cells. (*:p<0.001)
Figure IF represents a pie chart showing the percentage of origin sequences in genes sequences (light grey) and intergenic sequences (dark grey) in drosophila Kc cells. The value of gene association for randomized origins is indicated by the dashed pie (62%). (* :p<0.001)

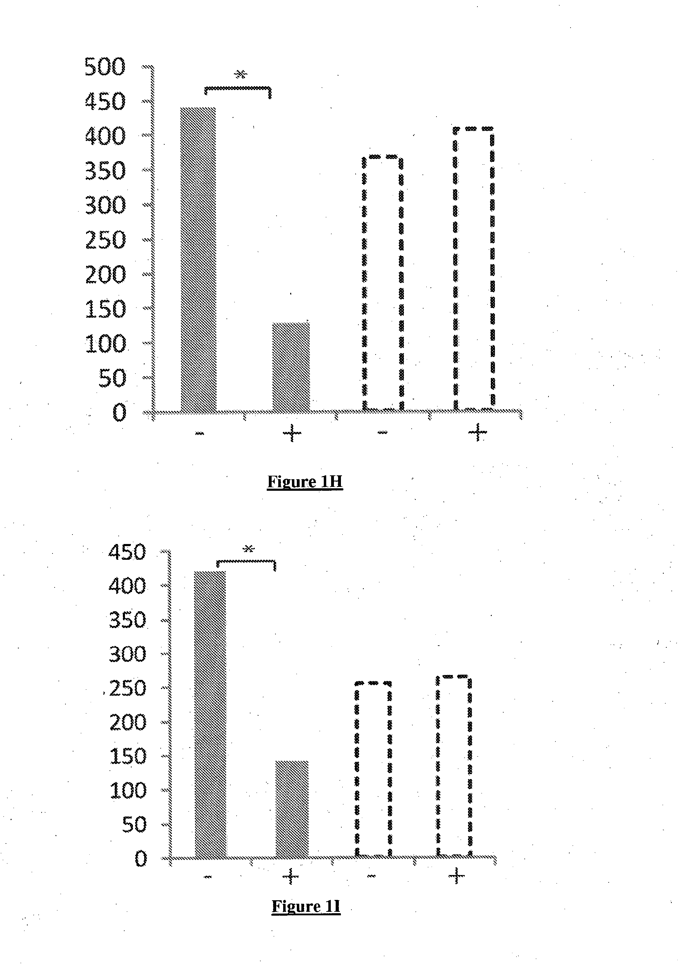

Figure 1G represents a graph showing the percentage of origin sequences in promotor sequences and intronic sequences and exonic sequences in drosophila Kc cells. The value of association for randomized origins is indicated by the dashed boxes. (* :p<0.001) Figure 1H represents a graph showing the association of replication origins with highly transcribed genes in MEF cells. The transcriptional output of gene associated ( +) or not (-) with replication origins is indicated. The average transcription of genes associated with randomly distributed origins is also shown. (*:p<0.001)
Figure 1I represents a graph showing the association of replication origins with highly transcribed genes in drosophila Kc cells. The transcriptional output of gene associated(+) or not (-) with replication origins is indicated. The average transcription of genes associated with randomly distributed origins is also shown. (*:p<0.001)

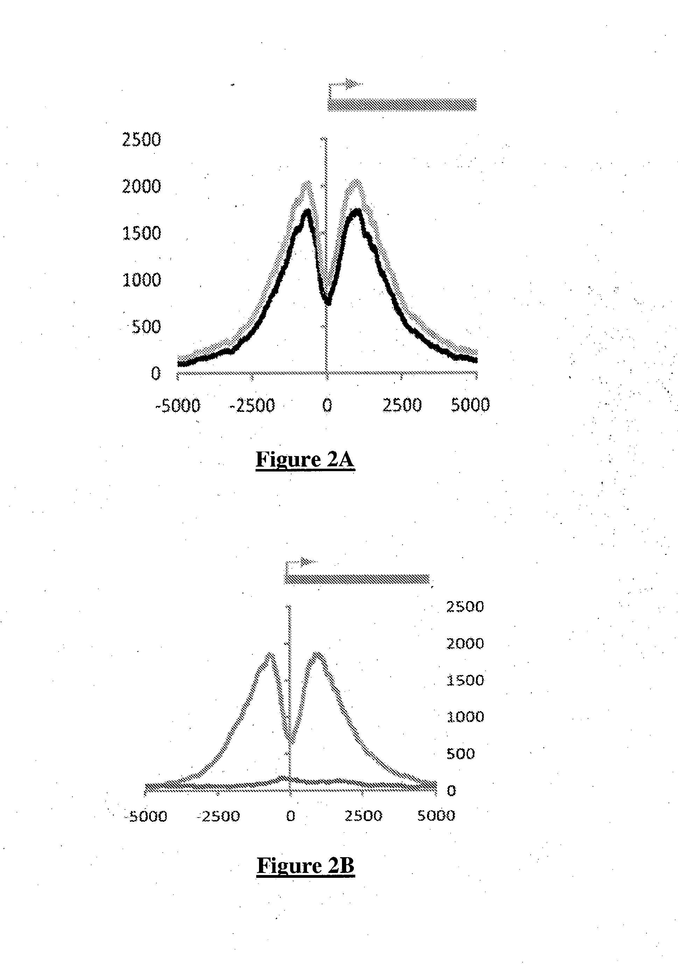

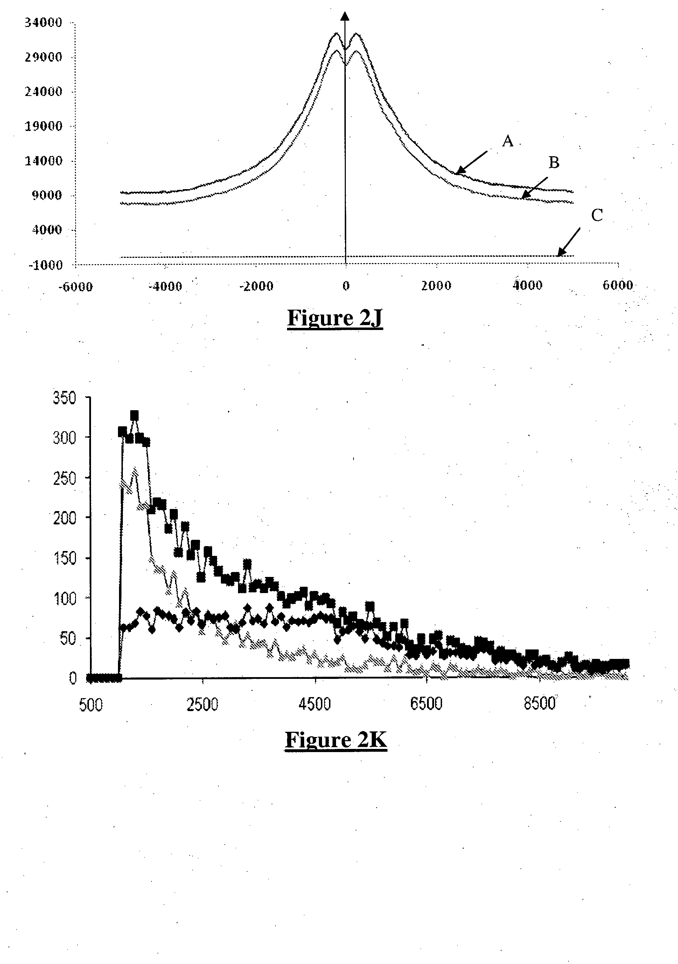

### Figures 2 A - K represent the association between CpG Islands and replication origins

Figure 2A represents the sum of all the Nascent Strands signals (corresponding to replication origins) around the site of initiation of transcription (TSS:Transcription Start Sites) in mouse MEF. Shown is the cumulative Nascent strand signal associated with all TSS (black line) and TSS associated with active replication origins (gray line).

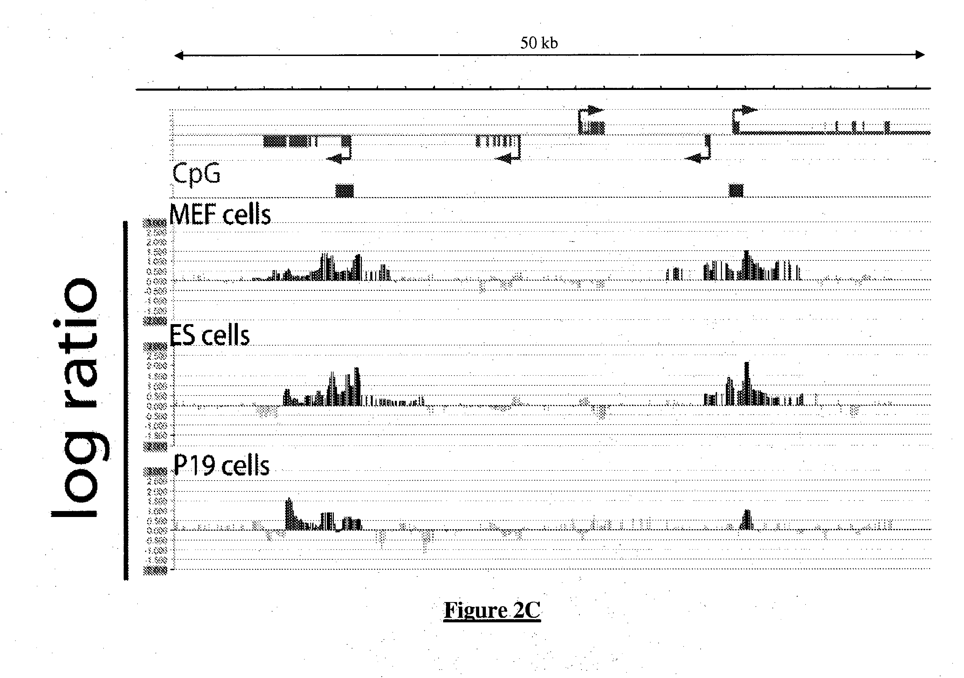

Figure 2B represents the sum of all the Nascent Strands signals around TSS associated with CpG Islands (CGI, light grey line) or not associated (dark grey line) in mouse MEF. Figure 2C represents an example of the association of replication origins of MEF, ES and P19 cells with CpG Islands. Shown is the localization of genes, CpG islands and Nascent Strands signals.

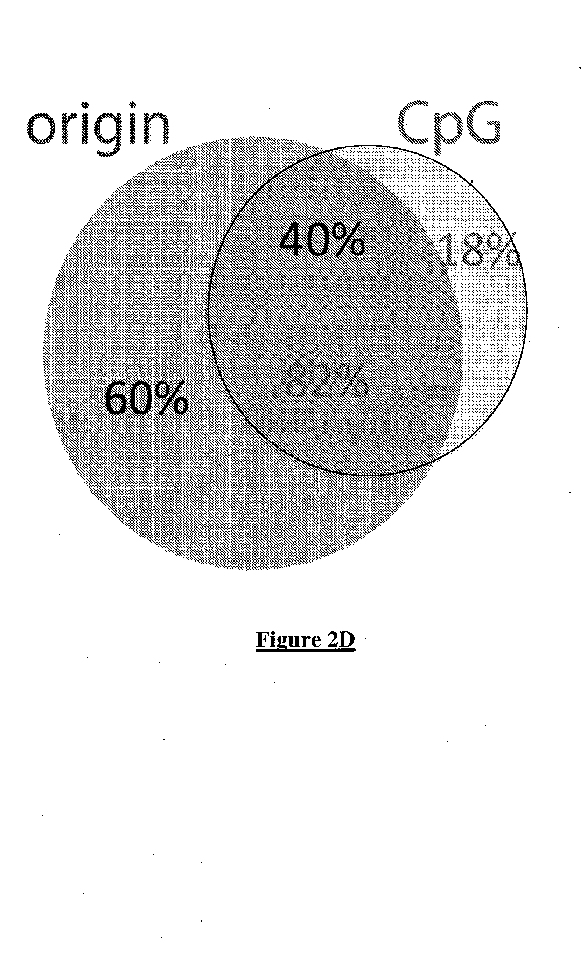

Figure 2D represents Venn diagram showing the strong association between replication origins and CpG Islands in mouse MEF. The percentage of association is indicated.

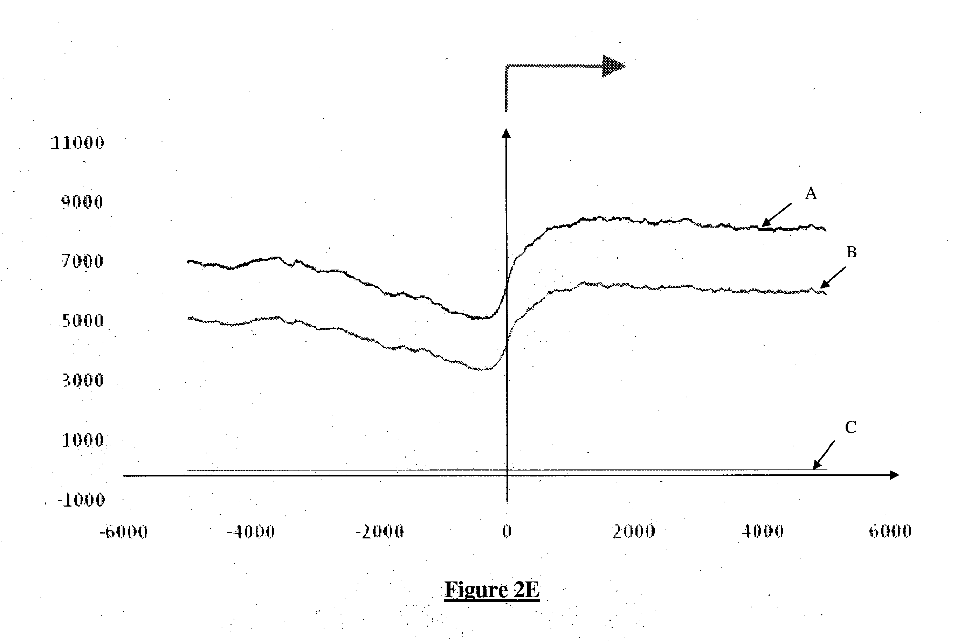

Figure 2E represents the sum of all the Nascent Strands signals (corresponding to replication origins) around the site of initiation of transcription (TSS:Transcription Start Sites) in drosophila Kc cells. Shown is the cumulative Nascent strand signal associated with all TSS (line 'b') and of TSS associated with active replication origins (line 'a') in proliferating cells. The cumulative signal of all TSS of mitotic and non-proliferating Kc cells is also shown (line 'c').

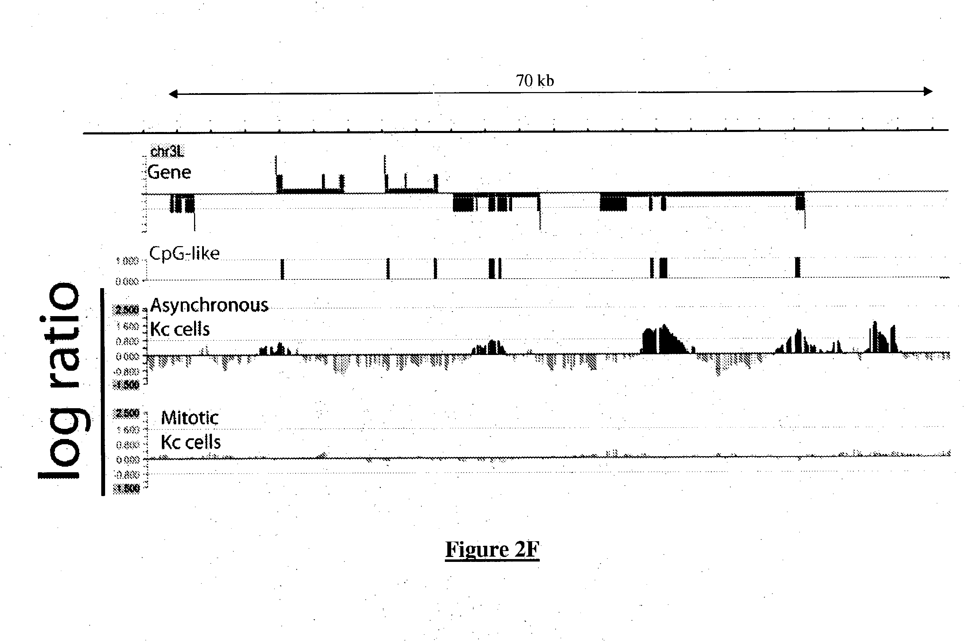

Figure 2F represents an example of the association of replication origins of drosophila Kc cells with CpG Islands-like sequences. Shown is the localisation of genes, CpG islands and Nascent Strands signals in proliferating and mitotic cells.

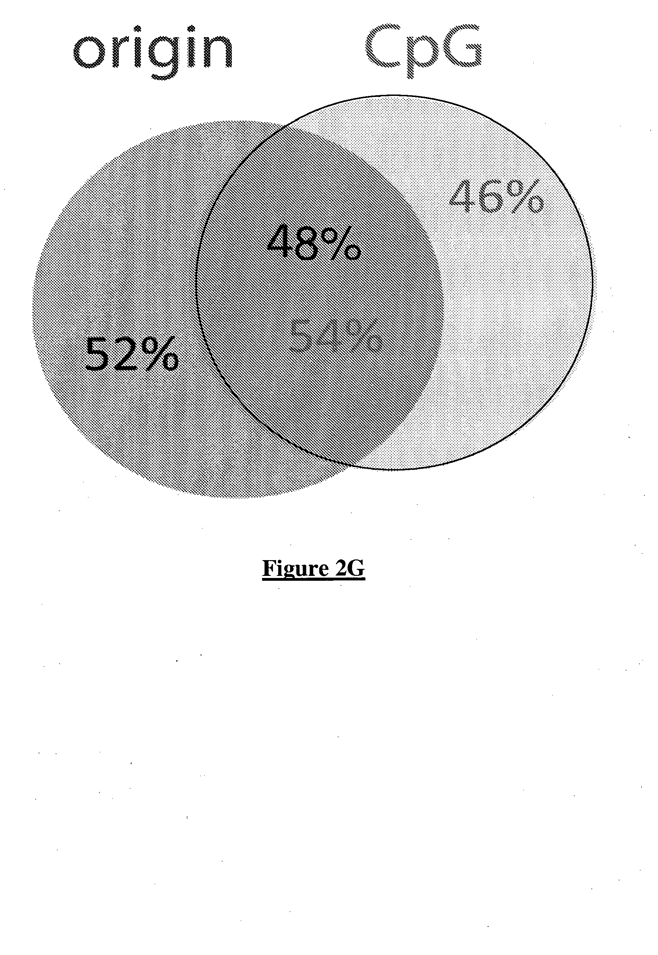

Figure 2G represents Venn diagram showing the strong association between replication origins and CpG Islands in drosophila Kc cells. The percentage of association is indicated.

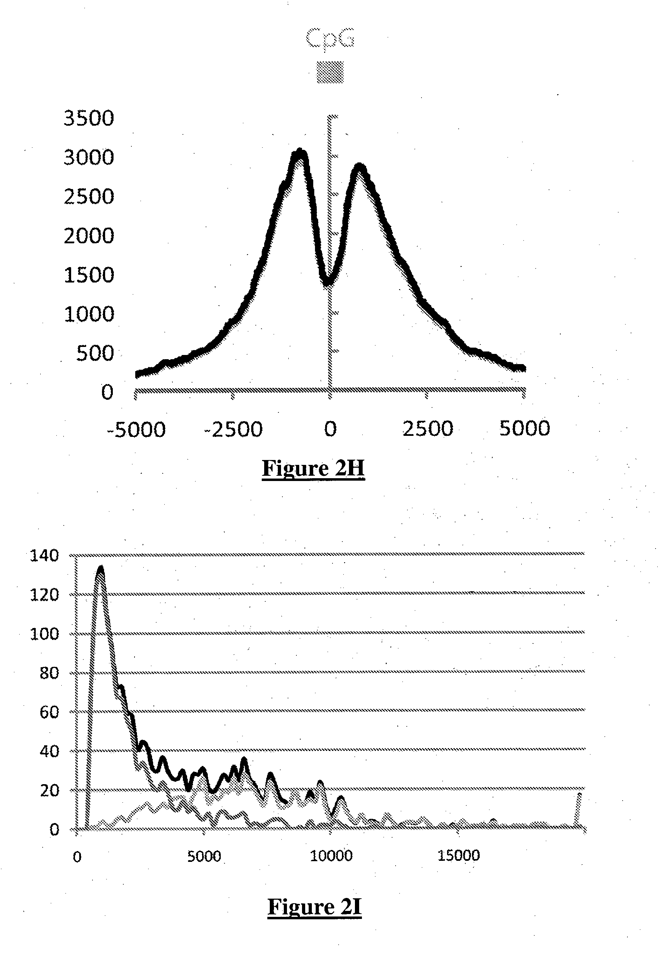

Figure 2H represents the sum of all the Nascent Strands signals ( corresponding to replication origins) around the CpG Islands in mouse MEF. Shown is the cumulative Nascent strand signal of all CpG Islands (grey line) and CpG Islands associated with active replication origins (black line).
Figure 2I represents the size of replication origins with regard to their association with CpG islands. The lines show the frequency of finding a replication origin of a particular length. All origins (black line) and origins associated (light grey) with CpG islands or not (dark grey line) in MEF are illustrated.
Figure 21 represents the sum of all the Nascent Strands signals (corresponding to replication origins) around the CpG Islands-like sequences in mouse MEF. Shown is the cumulative Nascent strand signal of all CpG Islands (line 'b') and of CpG Islands associated with active replication origins (line 'a') in proliferating cells. The cumulative signal of all CpG Islands of mitotic and non-proliferating Kc cells is also shown (line 'c').
Figure 2K represents the size of replication origins with regard to their association with CpG islands. The lines show the frequency of finding a replication origin of a particular length. All origins ('square' line) and origins associated ('diamond' line) with CpG islands or not ('triangle' line) in Kc cells are illustrated.

### Figures 3 A - J represent the common conserved motif in Metazoan replication origins.

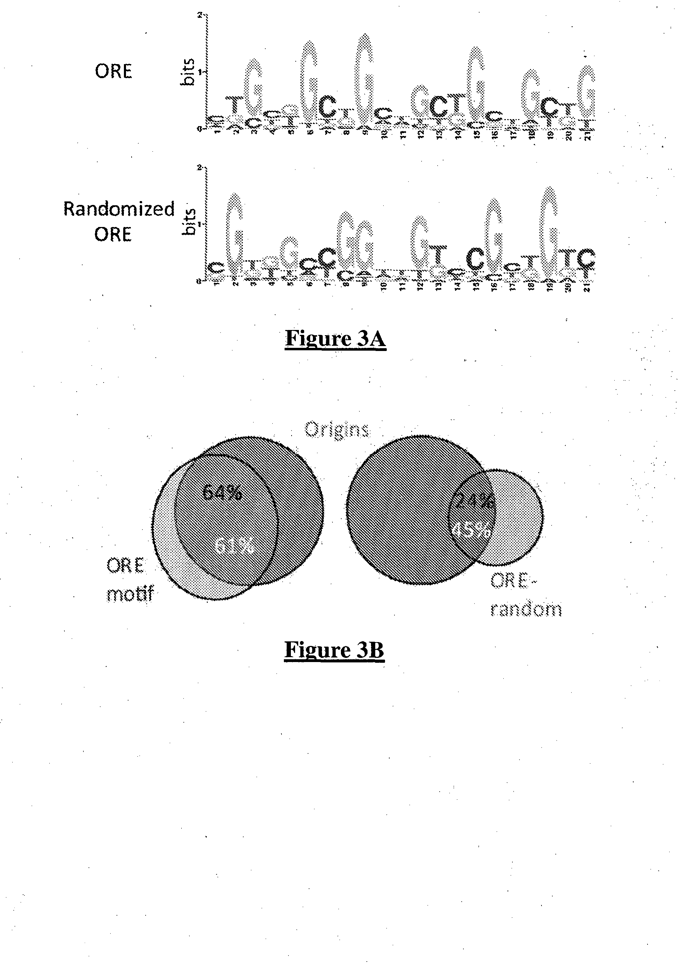

Figure 3A illustrates the consensus element found in metazoan replication origins. The 'ORE' (for Origin Repeated Element) motif was generated using MEME server withdrosophila origins. Also shown is a randomized motif to evaluate the specificity of the ORE. The size of letter represents the base preference for every position of the motif.
Figure 3B represents Venn diagram showing the strong association between replication origins and occurrences of the ORE in drosophila cells. The much weaker overlap between origins and the randomized motif is shown. The percentage of association is indicated.

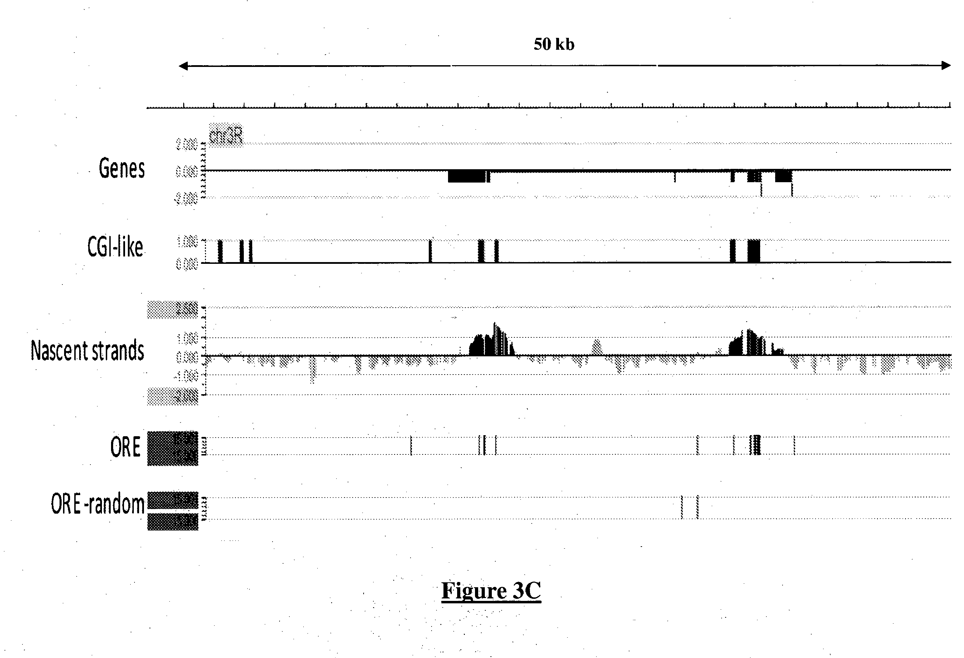

Figure 3C represents an example of the association of replication origins of Kc cells with occurrences of the ORE. Shown is the localization of genes, CpG islands-like sequences, Nascent Strands signals and occurrences of ORE and randomized ORE.

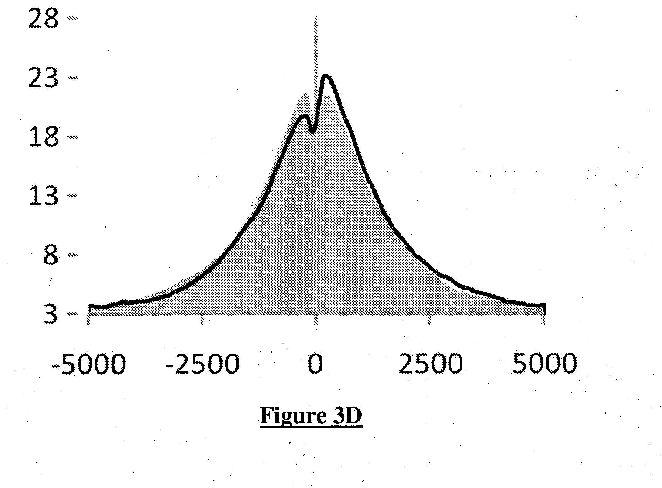

Figure 3D represents the sum of all the Nascent Strands signals ( corresponding to replication origins) around occurrences of the ORE in drosophila Kc cells. Shown is the cumulative Nascent strand signal associated with non-orientated motif (grey shadow) or with oriented ORE (black line). The x-axis represents the distance (in base pair) from ORE occurrences. The y-axis corresponds to cumulative p-value.

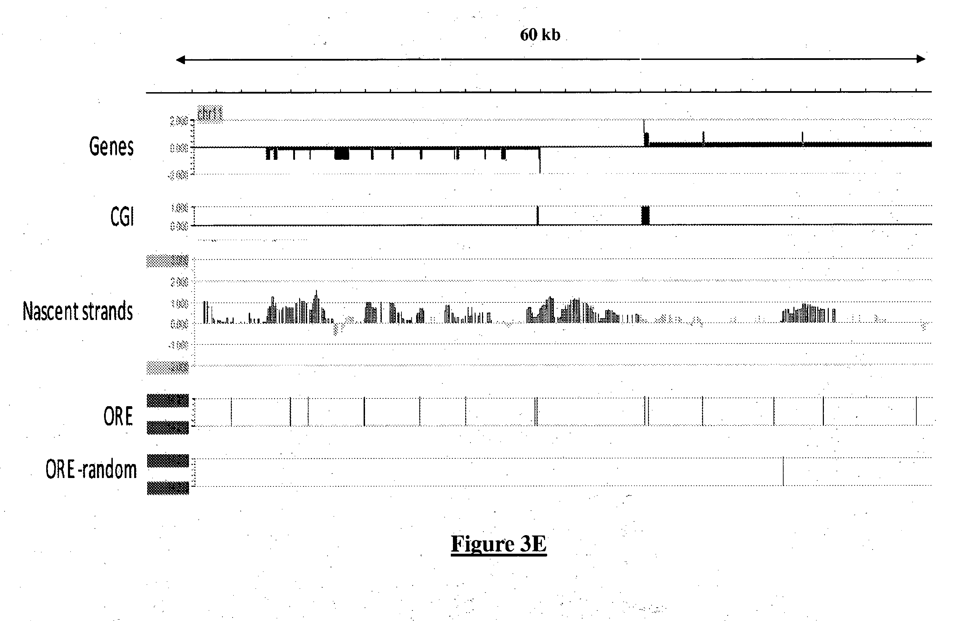

Figure 3E represents an example of the association of replication origins of P19 cells with occurrences of the ORE. Shown is the localization of genes, CpG islands, Nascent Strands signals and occurrences of ORE and randomized ORE.

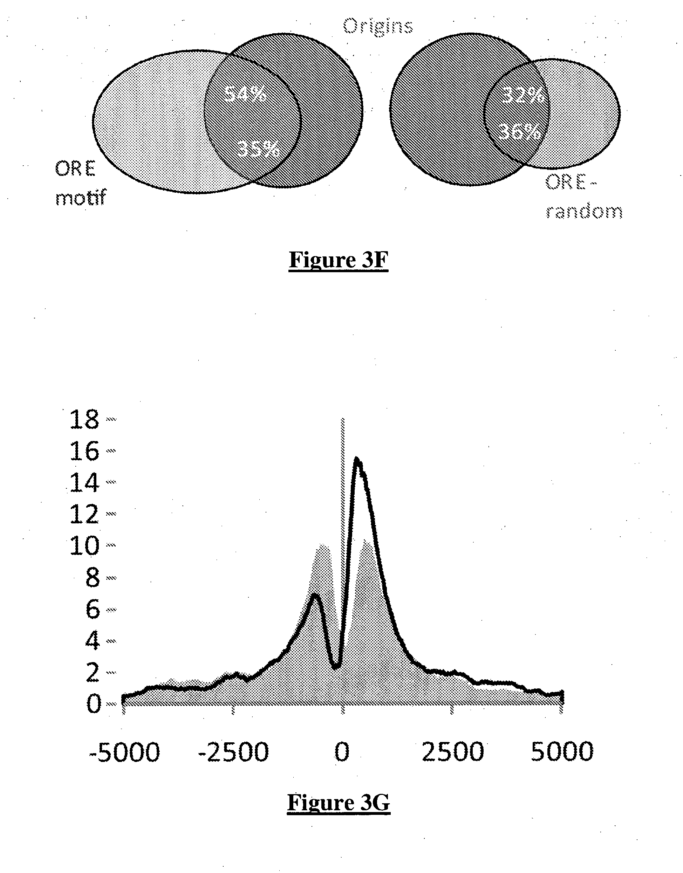

Figure 3F represents Venn diagram showing the strong association between replication origins and occurrences of the ORE in mouse MEP cells. The much weaker overlap between origins and the randomized motif is shown. The percentage of association is indicated.
Figure 3G represents the sum of all the Nascent Strands signals (corresponding to replication origins) around occurrences of the ORE in drosophila P19 cells. Shown is the cumulative Nascent strand signal associated with non-orientated motif (grey shadow) or with oriented ORE (black line). The x-axis represents the distance (in base pair) from ORE occurrences. The y-axis corresponds to cumulative p-value.
Figure 3H represents a graph showing the impact of varying the length of the ORE. Thelength of the motif is shown on the x-axis while the percentage of association is indicated on the y-axis. The percentage of association of occurrences of the ORE with origins (diamond) and the reciprocal relation (square) are shown.
Figure 3I shows the recognition of known human replication origins by the ORE. ORE occurrences and replication origins from the ENCODE project (Cadoret et al. 2008) are illustrated.
Figure 3J shows the recognition of the DHFR replication domain by the ORE. Genes, ORE occurrences and replication origins are illustrated.

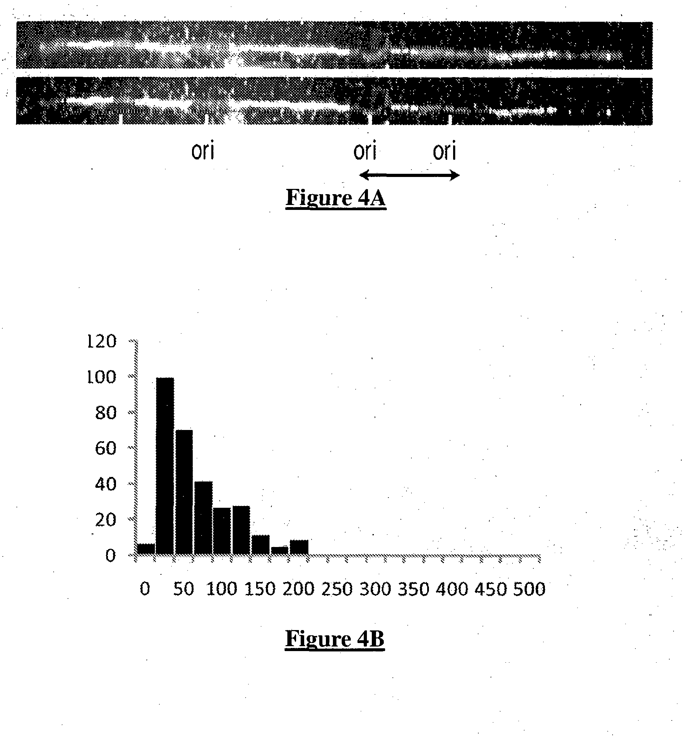

### Figures 4A - L represent the grouping into functional clusters along the chromosome of Metazoan replication origins.

Figure 4A shows an example of single-molecule analysis of the inter-origin spacing by molecular combing of DNA in Kc cells by two pulse labeling. The inferred position of replication origins is shown.
Figure 4B illustrates the distribution of the inter-origin distances in Kc cells. The x-axis represents the inter-origin spacing in kb while the frequency in shown on the y-axis.

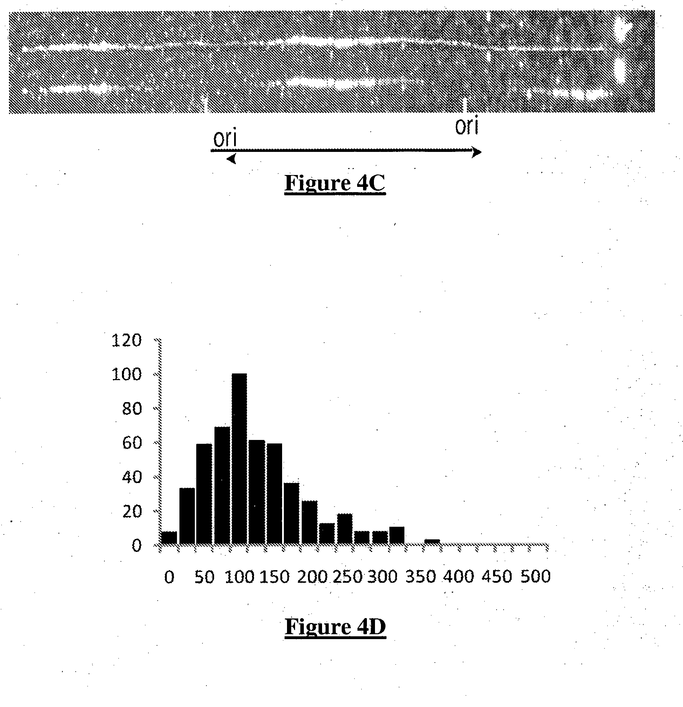

Figure 4C shows an example of single-molecule analysis of the inter-origin spacing by molecular combing of DNA in MEF cells by two pulse labeling. Very similar results were obtained for ES cells.
Figure 4D illustrates the distribution of the inter-origin distances in MEF cells. The x-taxis represents the inter-origin spacing in kb while the frequency in shown on the y-axis. Very similar results were obtained for ES cells.

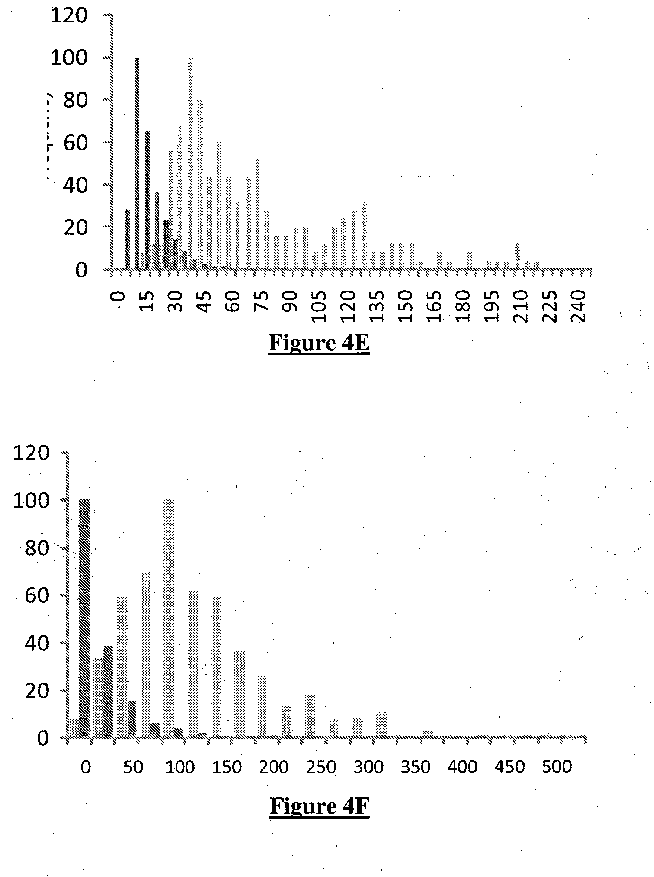

Figure 4E illustrates the distribution of the inter-origin distances obtained from combing data (grey bars) and from micro-array analysis (blue bars) in Kc cells. The x-axis represents the inter-origin spacing in kb while the frequency in shown on the y-axis. Figure 4F illustrates the distribution of the inter-origin distances obtained from combing data (grey bars) and from micro-array analysis (blue bars) in MEF cells. The x-axis represents the inter-origin spacing in kb while the frequency in shown on the y-axis. Very similar results were obtained for ES cells.

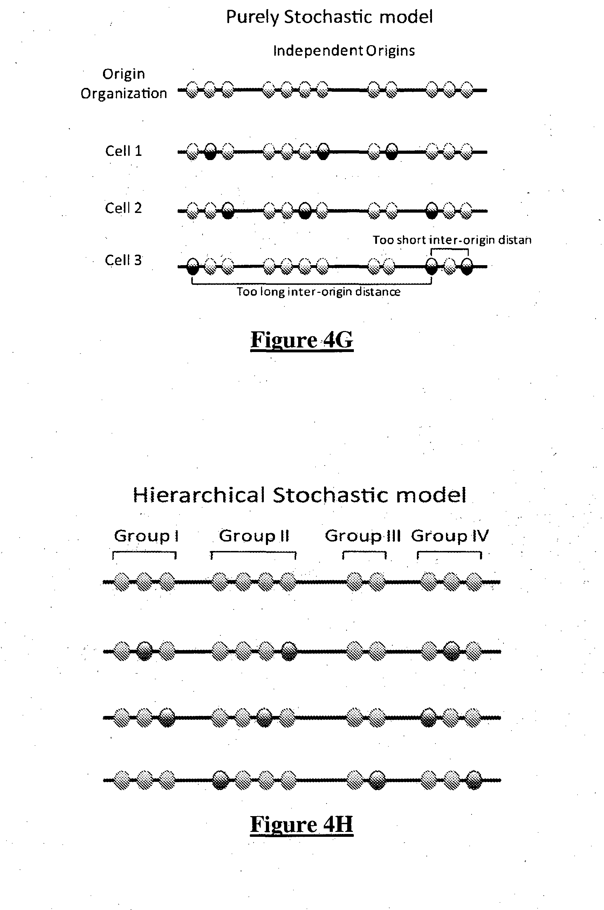

Figure 4G illustrates the Purely Stochastic Model of Ori firing. In this model, Oris are completely independ**e**nt and are activated randomly (red cercles). Very short and long inter-origin distances are observed.
Figure 4H illustrates the Hierarchical Stochastic Model. In this model, Oris are linked within functional units where activation of one Ori silences the others in the same group.

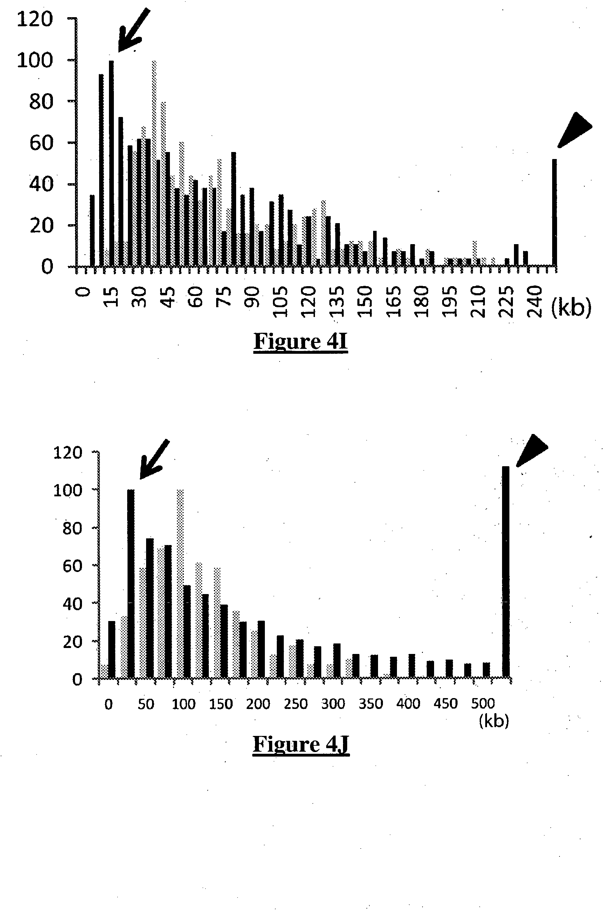

Figure 4I shows the distribution of the inter-origin distances obtained from combing data of Kc cells (light grey bars) and from computational simulations ( dark grey bars). In the tested model, replication origins were picked at random. Note the presence of short (arrow) and long (arrowhead) inter-origin distances in the simulated dataset not found in the combing analysis. The x-axis represents the inter-origin spacing in kb while the frequency in shown on the y-axis.
Figure 4J shows the distribution of the inter-origin distances obtained from combing data of MEF cells (light grey bars) and from computational simulations (dark grey bars). In the tested model, replication origins were picked at random. Note the presence of short (arrow) and long (arrowhead) inter-origin distances in the simulated dataset not found in the combing analysis. The x-axis represents the inter-origin spacing in kb while the frequency in shown on the y-axis. Very similar results were obtained for ES cells.

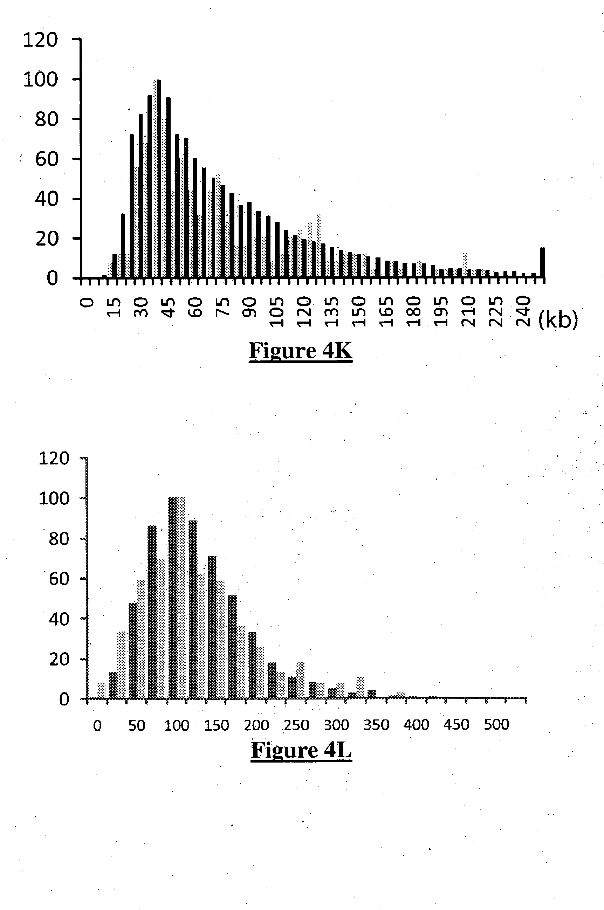

Figure 4K shows the distribution of the inter-origin distances obtained from combing data of Kc cells (light grey bars) and from computational simulations (light grey bars). In the tested model, replication origins are clustered into functional groups where the firing of one randomly chosen replication origin suppresses the activation of the other origins within the same group. Both set of data correlate well. The x-axis represents the inter¬origin spacing in kb while the frequency in shown on the y-axis.
Figure 4L shows the distribution of the inter-origin distances obtained from combing data of MEF cells (light grey bars) and from computational simulations (light grey bars). In the tested model, replication origins are clustered into functional groups where the firing of one randomly chosen replication origin suppresses the activation of the other origins within the same group. Both set of data correlate well. The x-axis represents the inter-origin spacing in kb while the frequency in shown on the y-axis. Very similar results were obtained for ES cells.

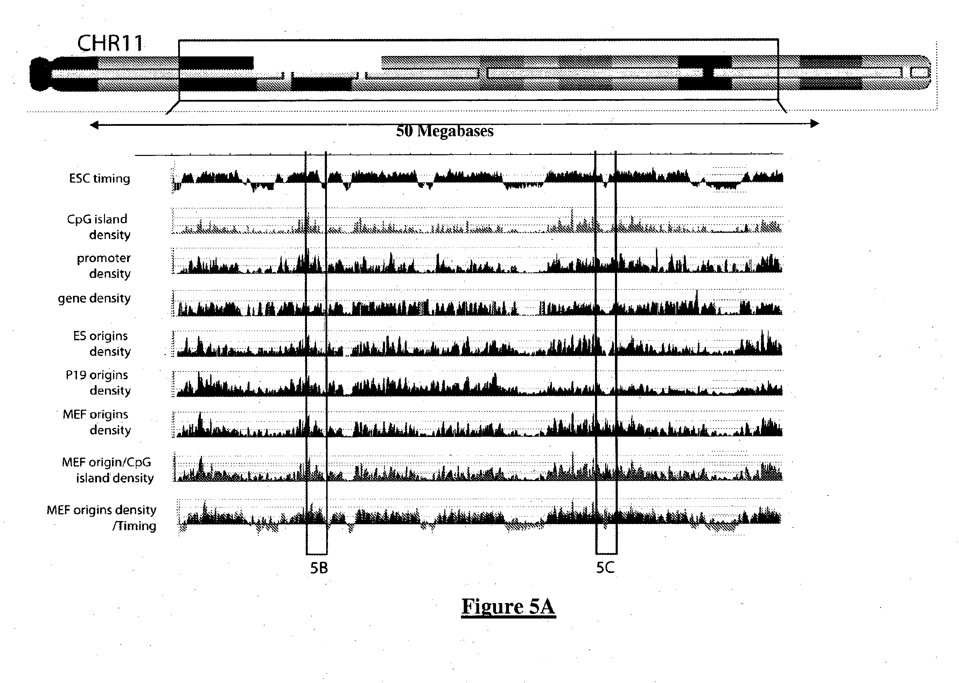

### Figures 5 A - D represent the domains of origin density correlated with domains of CpG island density and replication timing

Figure 5A represents the totality of the 60,S MB on the region defined for the mouse chromosome 11. Diagrams show the replication timing, CpG island density, exon and gene density and replication origins density for mouse cells. The panels below represent the significant overlay of MEF origins and CpG or replication timing domains. The region analyzed in Figure SB and SC are highlighted.
Figure SB represents a 3.S Mb region of mouse chromosome 11. Note that all indicators are relatively high in this early replication region as defined in ES cells.

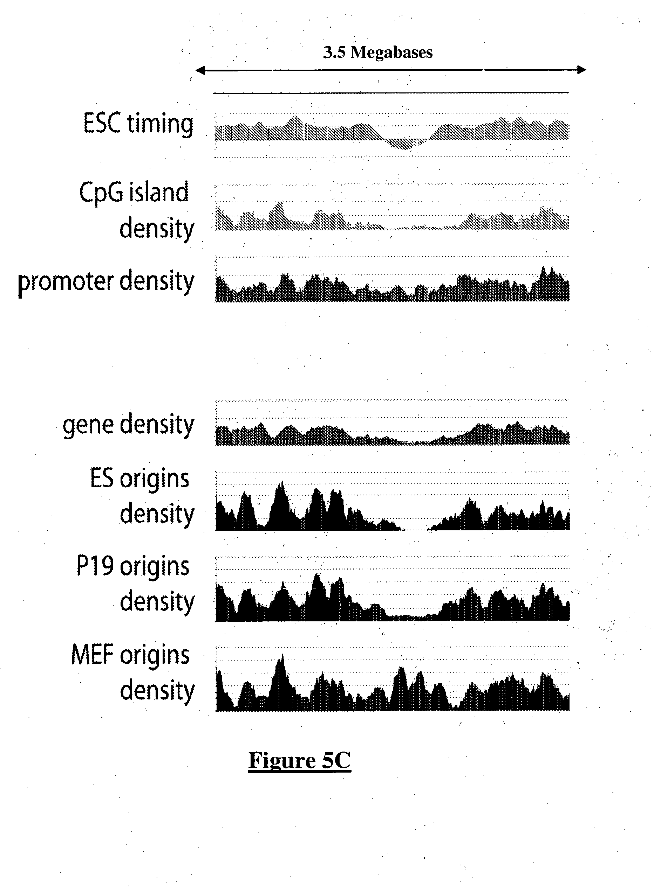

Figure 5C represents a 3 .5 Mb region of mouse chromosome 11. Note the differences in origin density between MEF and pluripotent cells in the late replicating domain.

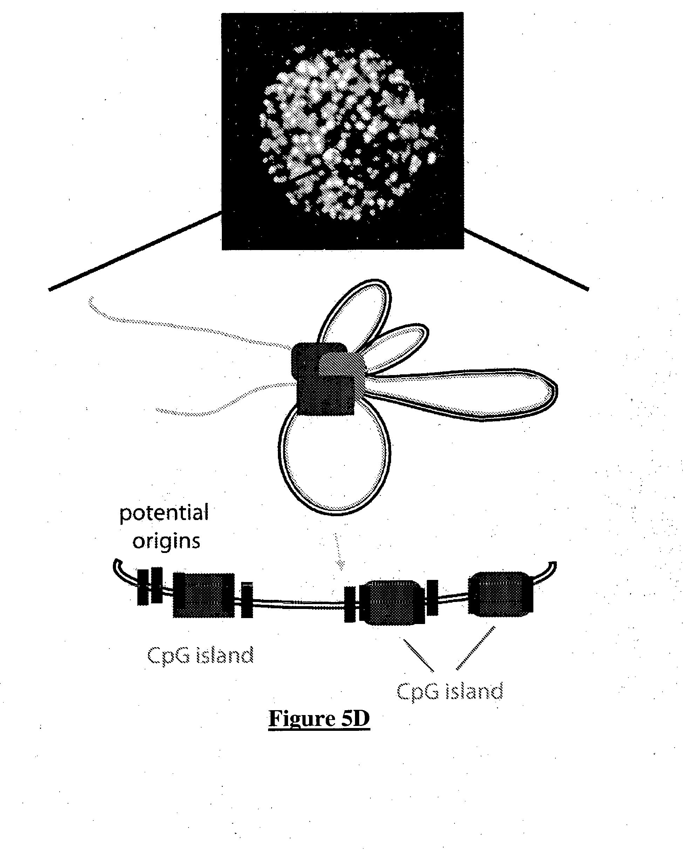

Figure 5D shows a model illustrating genomic distribution and usage of replication origins in metazoan. Multiple loops could cluster several fired replication origins in foci. For illustration purposes, BrdU positive replication foci are shown (top panel). CpG Island could be a regulatory element for location and for efficiency firing of replication origins.
In this model one origin by cluster can be fired in each cell.

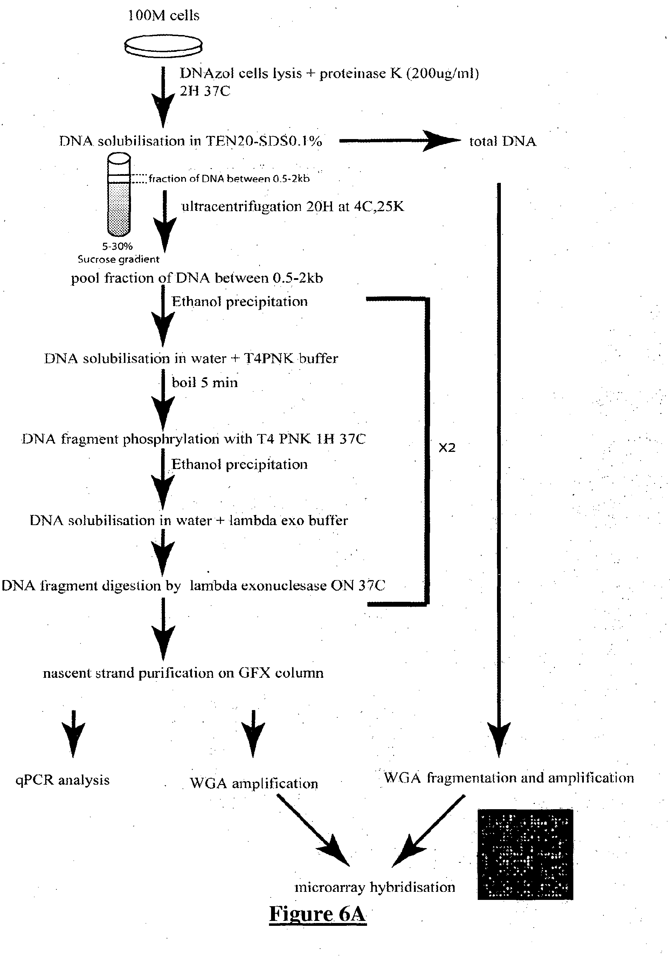

### Figures 6 A - E represent the purification process of Nascent Strands DNA from cultured cells.

Figure 6A shows the scheme used for the purification and the analysis of metazoan replication origins.

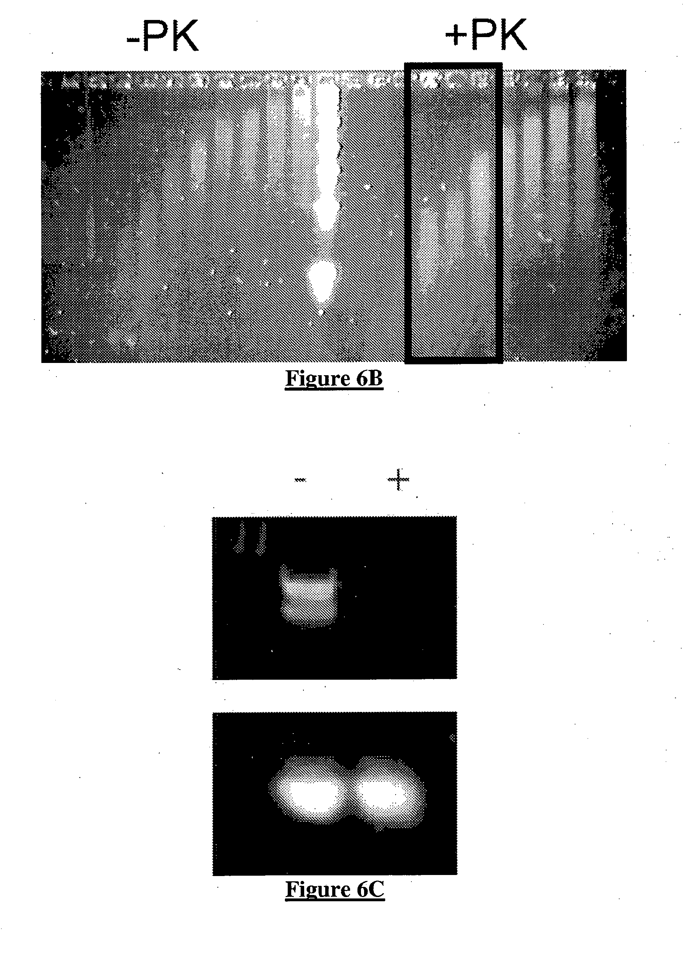

Figure 6B shows the analysis of the fraction obtained after the sucrose ultracentrifugation step. Fractions were analyzed by alkaline agarose gel electrophoresis. In this particular experiment, proteinase K (PK) was added(+) or not(-) during lysis. Fractions of 0.5-2 kb' DNA are pooled (black box) for the following step.
Figure 6C illustrates the specificity of lambda exonuclase. DNA (upper panel) or RNA (lower panel) samples were incubated with ( +) or without (-) lambda exonuclease. The reaction was separated by agarose gel electrophoresis and visualized using GelRed staining.

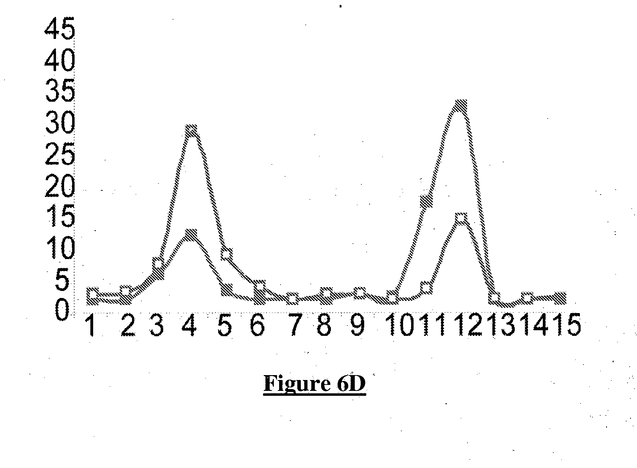

Figure 6D illustrates the effect of our amplification protocol. Shown is the qPCR analysis of the HoxA locus in P19 cells of the un-amplified Nascent Strands sample (empty square) and the WGA-amplified Nascent Strands (filled square). The x-axis identify the primer used in the qPCR analysis while the y-axis represents the fold enrichment of Nascent Strands compared to negative primers.

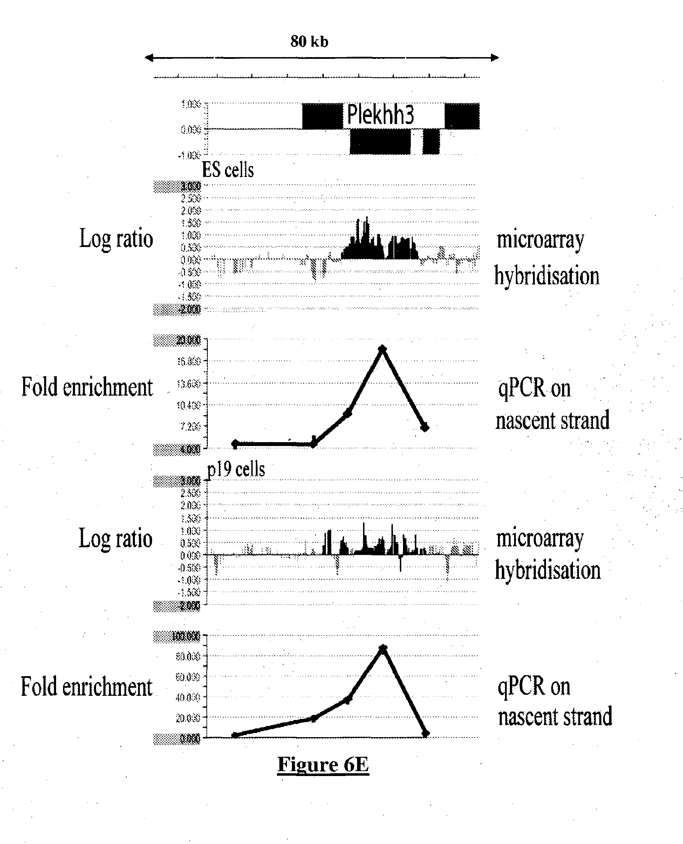

Figure 6E shows that Nascent Strands signals from microarrays can be observed by qPCR in mouse P19 and ES cells. Genes localization, Nascent Strands signals and qPCR analysis are shown.

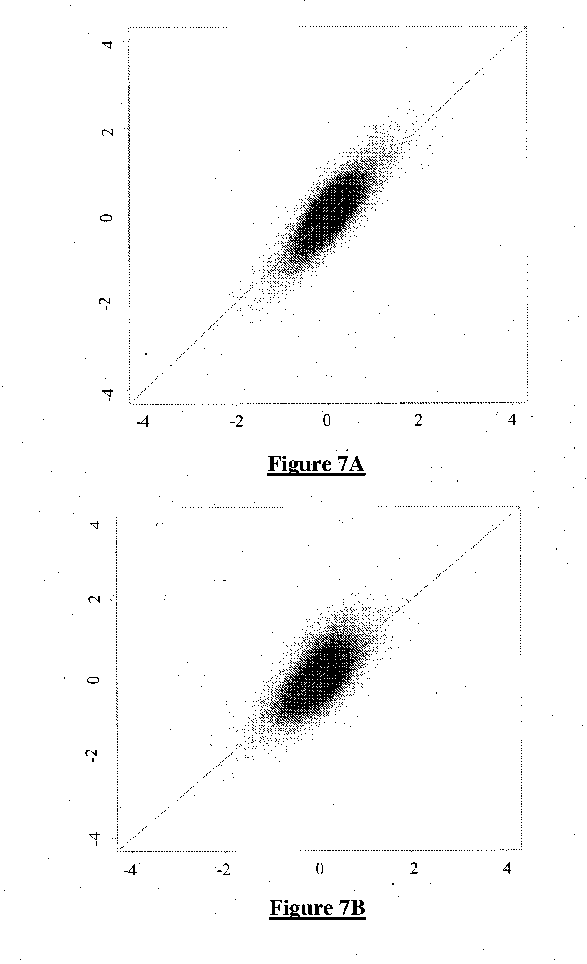

### Figures 7 A-C represent the reproducibility of Nascent Strands purification.

Figure 7A show scatter plots comparing two biological replicates of purified Nascent Strands from P19 cells. Every dot represents a single probe on the microarray. Its position is determined by the value of the log ratio of the two compared replicates. The coefficient of determination (R2) is 0.7935912.
Figure 7B show scatter plots comparing two biological replicates of purified Nascent Strands from Kc cells. Every dot represents a single probe on the microarray. Its position is determined by the value of the log ratio of the two compared replicates. The coefficient of determination (R2) is 0.7057634.

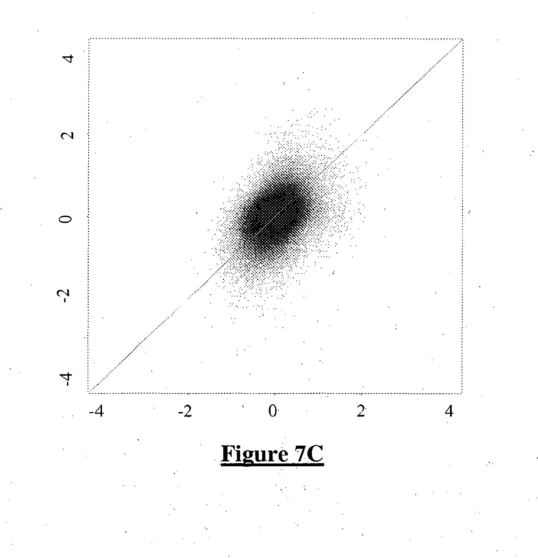

Figure 7C show scatter plots comparing two biological replicates of purified Nascent Strands from ES cells. Every dot represents a single probe on the microarray. Its position is determined by the value of the log ratio of the two compared replicates. The coefficient of determination (R2) is 0.3724884.

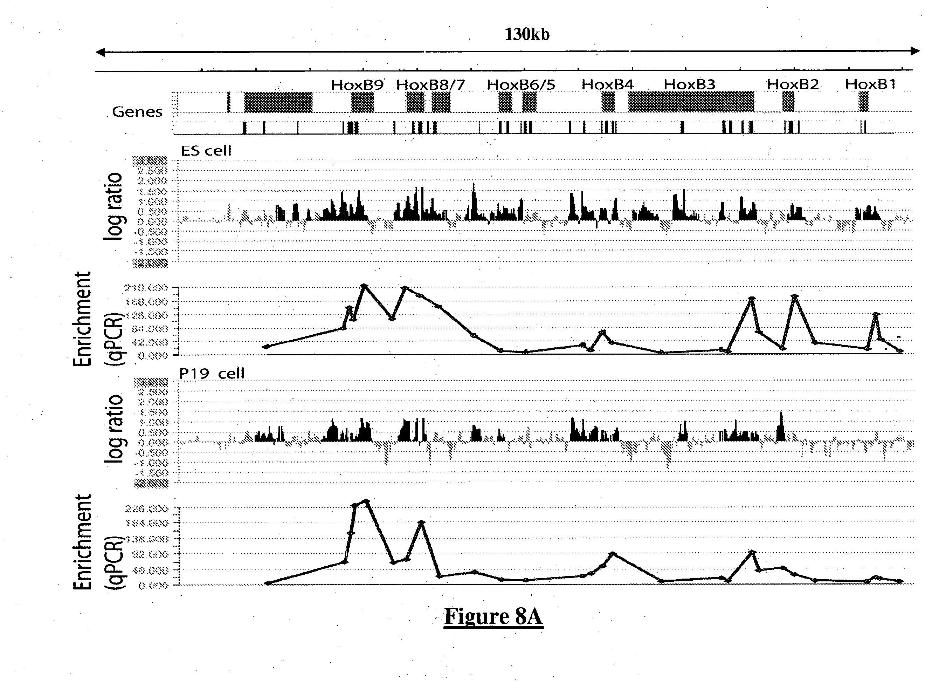

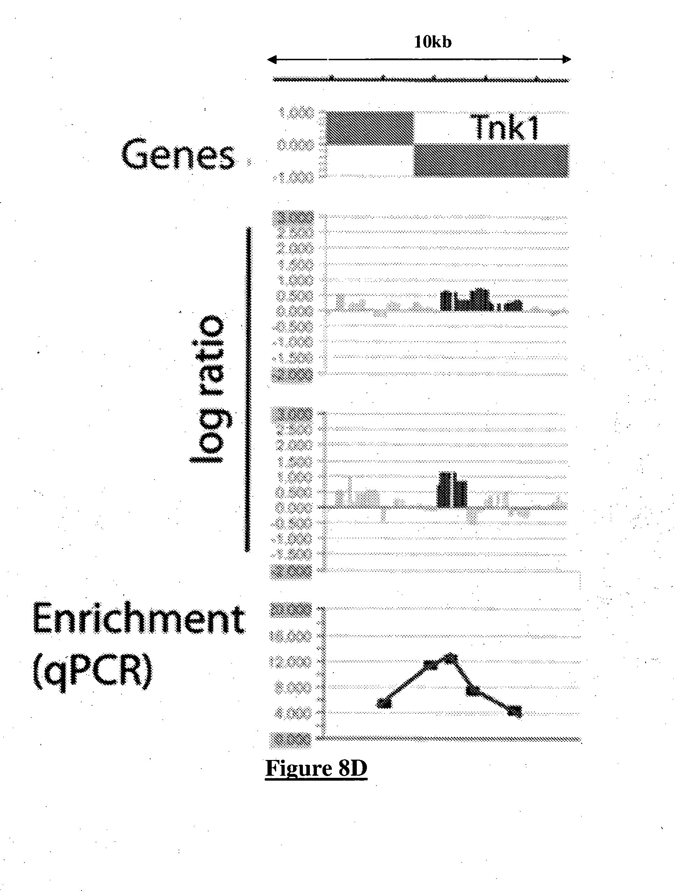

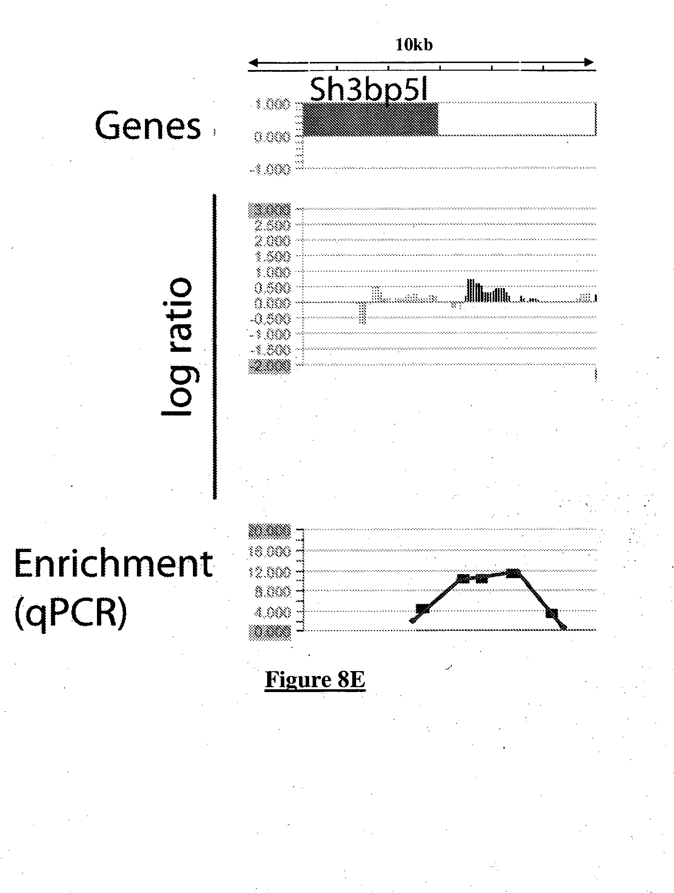

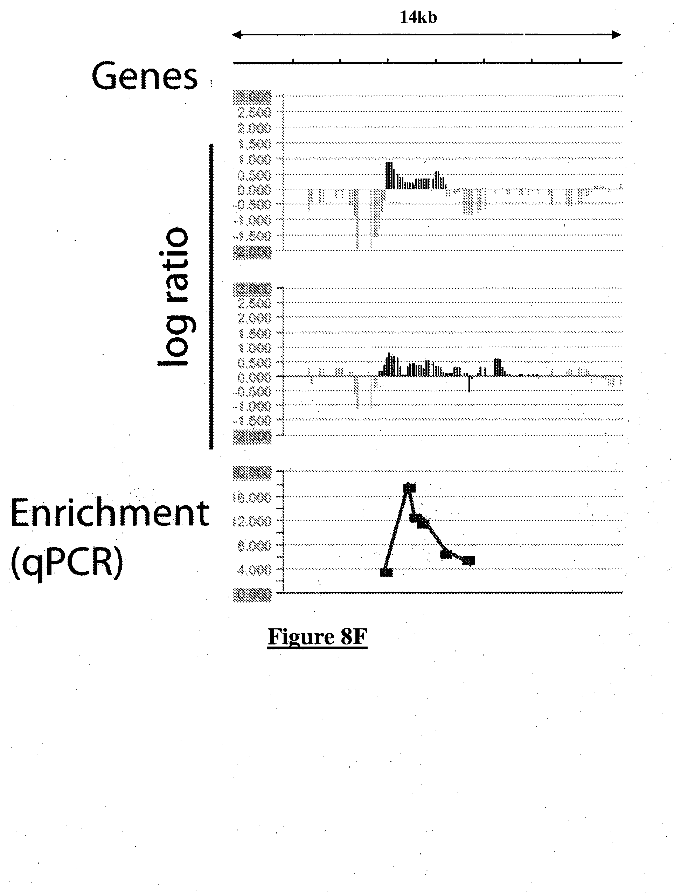

### Figures 8 A- F represent the confirmation using qPCR analysis of replication origins identified by microarrays.

Figure SA represents replication origins analysis of the LoxB locus. Shown is the localization of genes, the Nascent Strands signals from microarray analysis and qPCR analysis for ES and P 19 cells.
Figure SB shows that our Nascent Strands preparation contains a known origin. Represented is a qPCR analysis of the replication origin of e-mye gene.

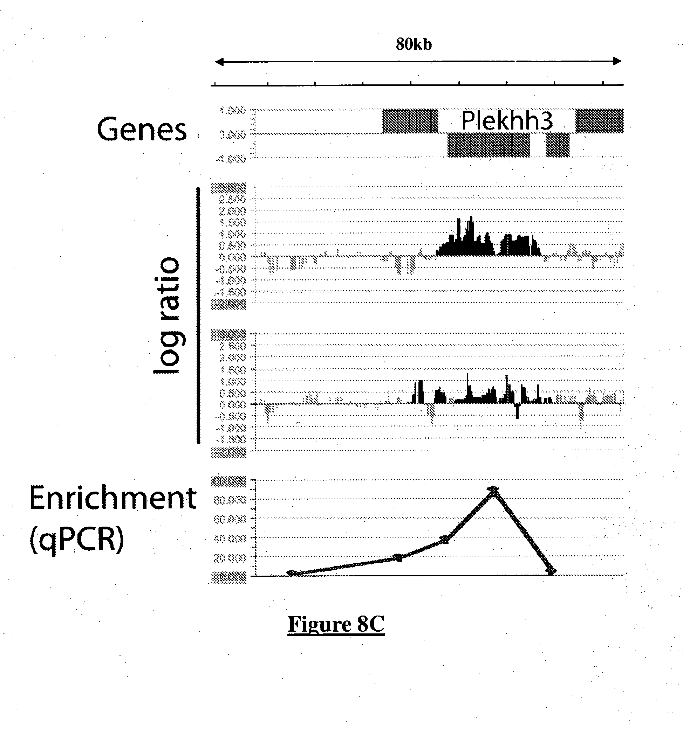

Figures 8C-8F show that novel replication origins identified in our microarrays can be observed by qPCR in mouse P19 and ES cells. Genes localization, Nascent Strands signals and qPCR analysis are shown. In Figure SC, SD and 8F, the upper panel of microarray data is for ES cells while the lower panel is for P19 cells. In Figure SE, results for ES cells are shown.

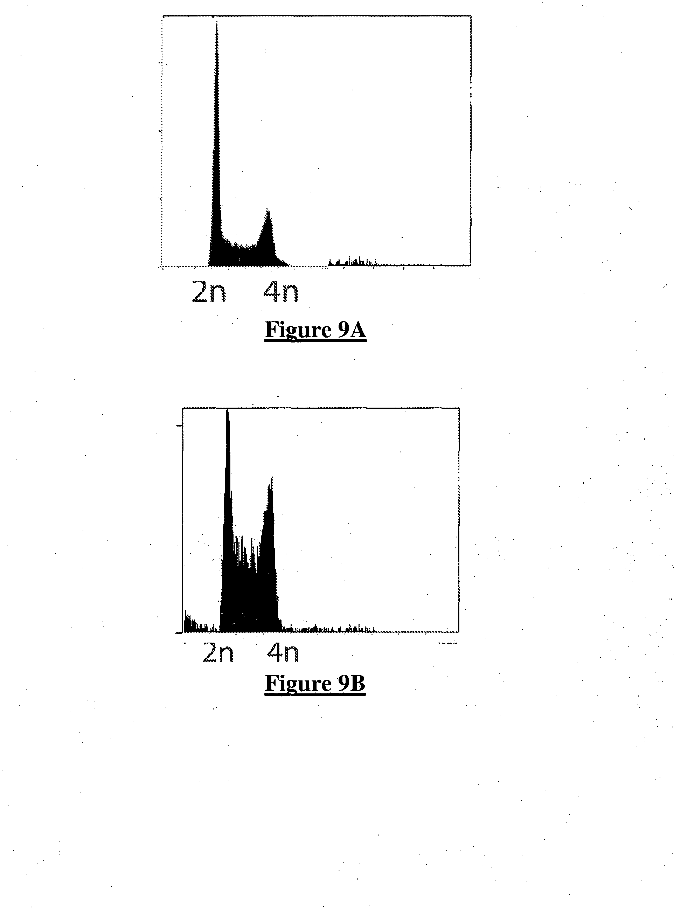

### Figures 9A-F represent the cell cycle distribution of cells used for the Nascent

Strands purifications. The DNA content of individual cells is stained and quantified using a flow cytometer. The populations of cells before (2n) and after (4n) DNA replication are indicated. Cells in between 2n and 4n are replicating DNA.

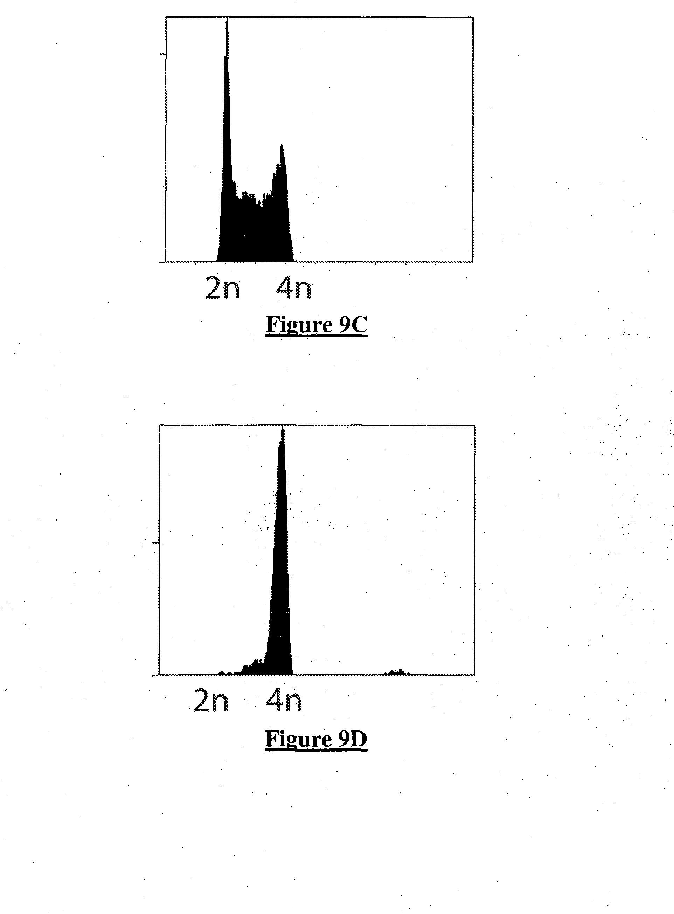

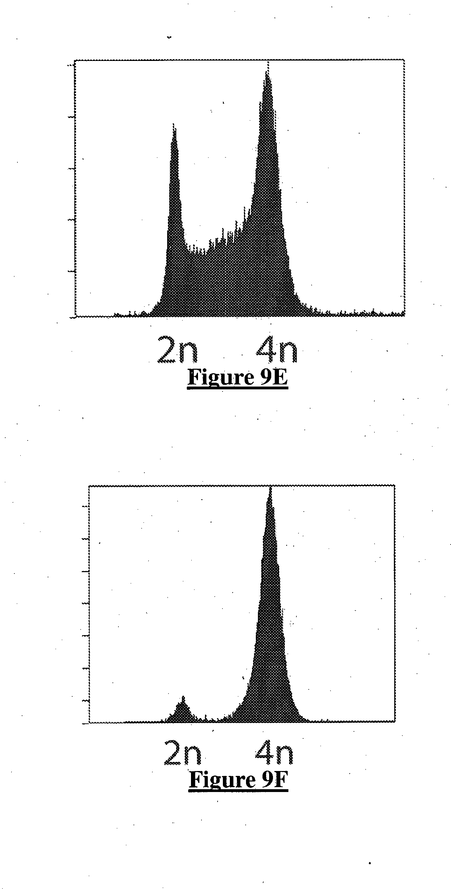

Figure 9A represents DNA content ofMEF cells actively proliferating Figure 9B represents DNA content of ES cells actively proliferating. Figure 9C represents DNA content of P19 cells actively proliferating. Figure 9D represents DNA content of P19 cells arrested in mitosis. Figure 9E represents DNA content of Kc cells actively proliferating.
Figure 9F represents DNA content of Kc cells arrested in mitosis.

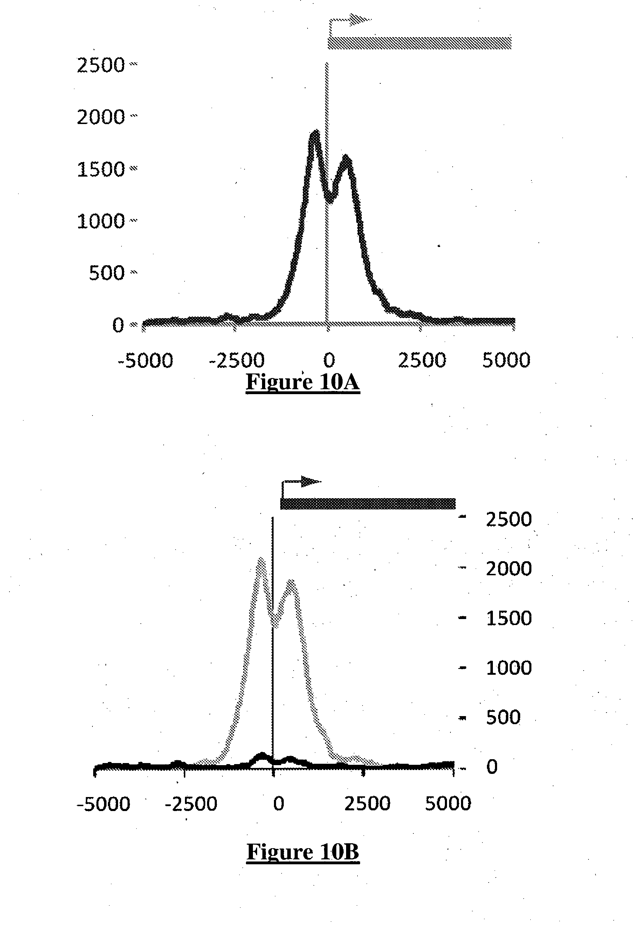

### Figures 10 A - H represent the association between CpG Islands and replication origins in ES and P19 cells.

Figure 10A represents the sum of all the Nascent Strands signals (corresponding to replication origins) around the site of initiation of transcription (TSS: Transcription Start Sites) in mouse ES cells. Shown is the cumulative Nascent Strands signals associated with all TSS.
Figure 10B represents the sum of all the Nascent Strands signals around TSS associated with CpG Islands (CGI, light grey line) or not associated (dark grey line) in mouse ES cells.

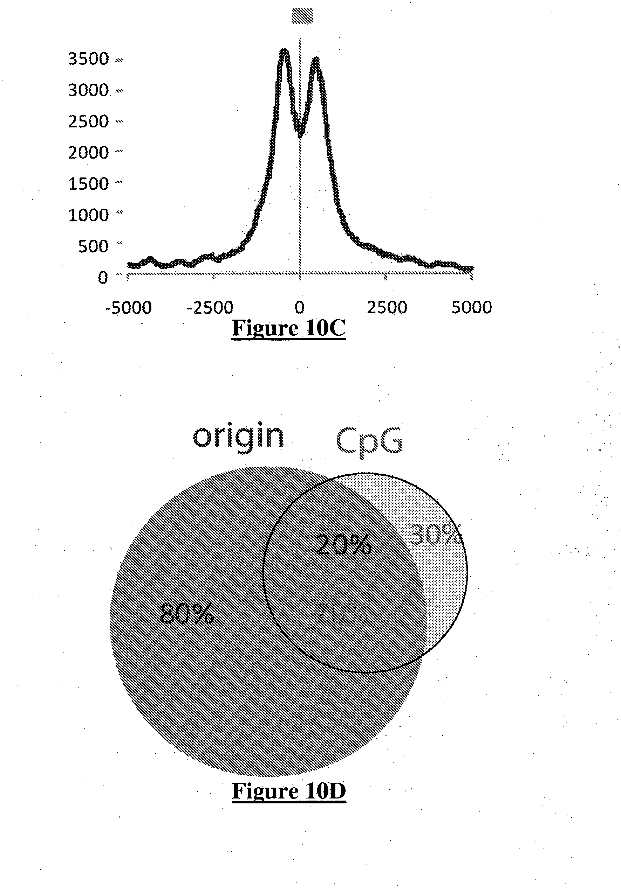

Figure 10C represents the sum of all the Nascent Strands signals (corresponding to replication origins) around the CpG Islands in mouse ES cells. Shown are the cumulative Nascent Strands signals of all CpG Islands.
Figure 10D represents Venn diagram showing the strong association between replication origins and CpG Islands in mouse ES cells. The percentage of association is indicated.

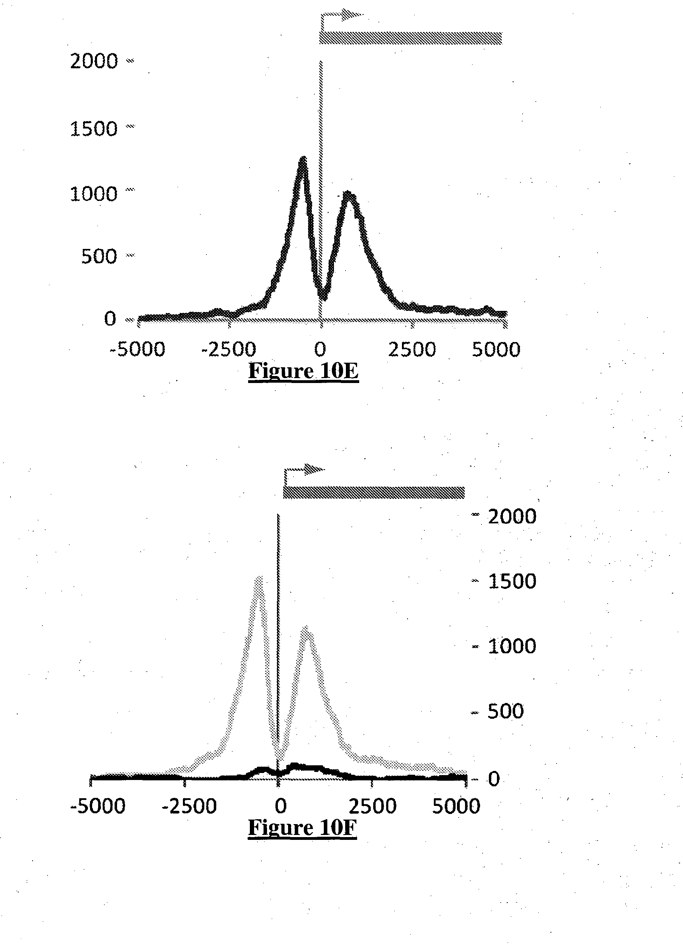

Figure 10E represents the sum of all the Nascent Strands signals (corresponding to replication origins) around the site of all initiation of transcription (TSS:Transcription Start Sites) in mouse P19 cells.
Figure 10F represents the sum of all the Nascent Strands signals around TSS associated with CpG Islands (CGI, light grey line) or not associated (dark grey line) in mouse P19 cells.

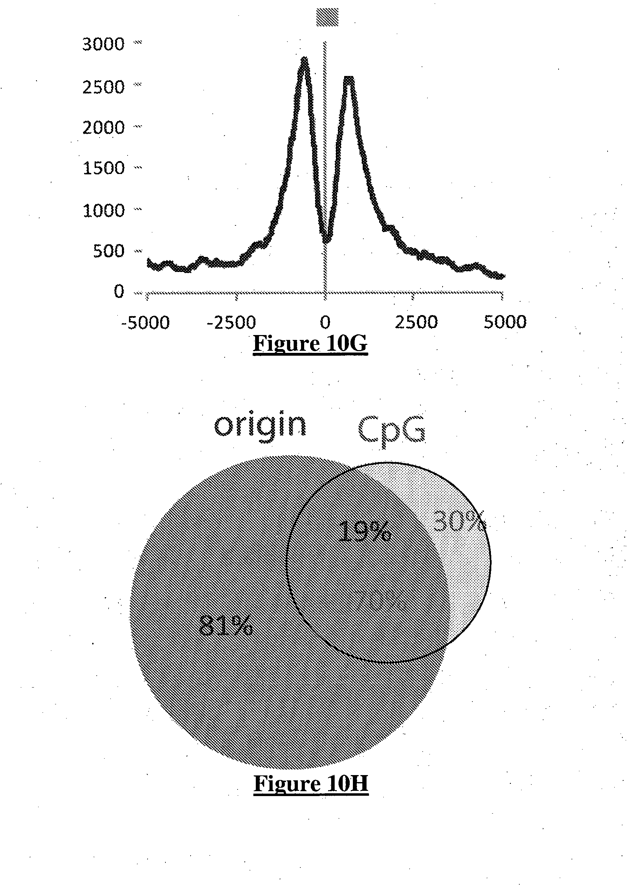

Figure 10G represents the sum of all the Nascent Strands signals ( corresponding to replication origins) around the CpG Islands in mouse P 19 cells. Shown are the cumulative Nascent Strands signals of all CpG Islands.
Figure 10H represents Venn diagram showing the strong association between replication origins and CpG Islands in mouse P 19 cells. The percentage of association is indicated.

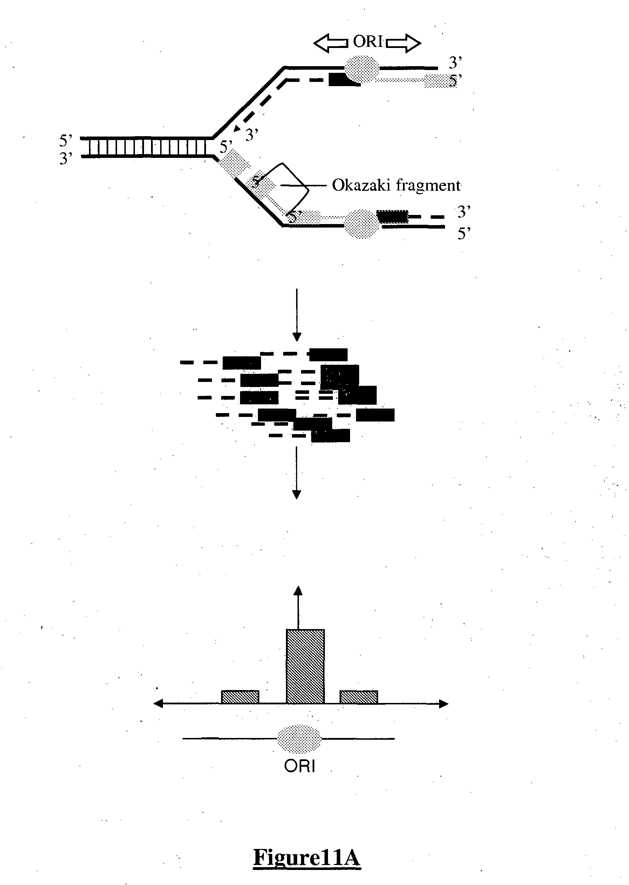

### Figure 11 A - B correspond to a schematics representations of the Replication origins mapping by nascent strands relative enrichment assay.

Figure 11A is a schematic representation of the process: Nascent strands are purified and then analyzed by qPCR. Brocken lines represent nascent DNA, black boxes represent RNA primers.

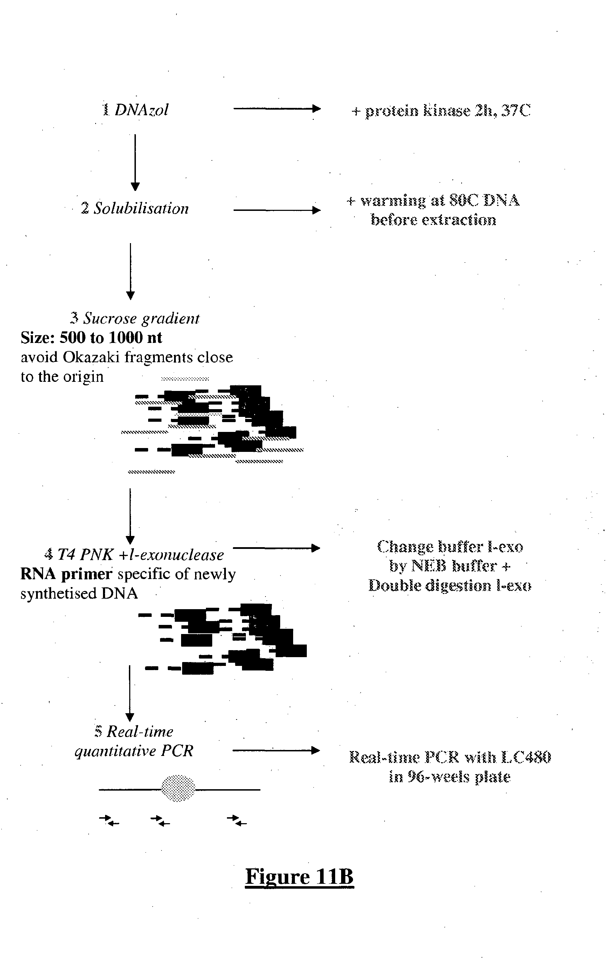

Figure 11B represents the detailed process. Cells are first lysed in the DNAzol then purified and total DNA is heated and placed on a sucrose gradient. The sucrose fractions containing DNA fragments of interest between 500 and 2000 base pairs are once phosphorylated by T4 polynucleotide kinase and then digested by lambda exonuclease. After extraction by phenol-chloroform, DNA remaining was again treated with T4 PNK and lambda exonuclease. Purified nascent strands are analyzed by qPCR. Grey lines represent contaminant DNA.

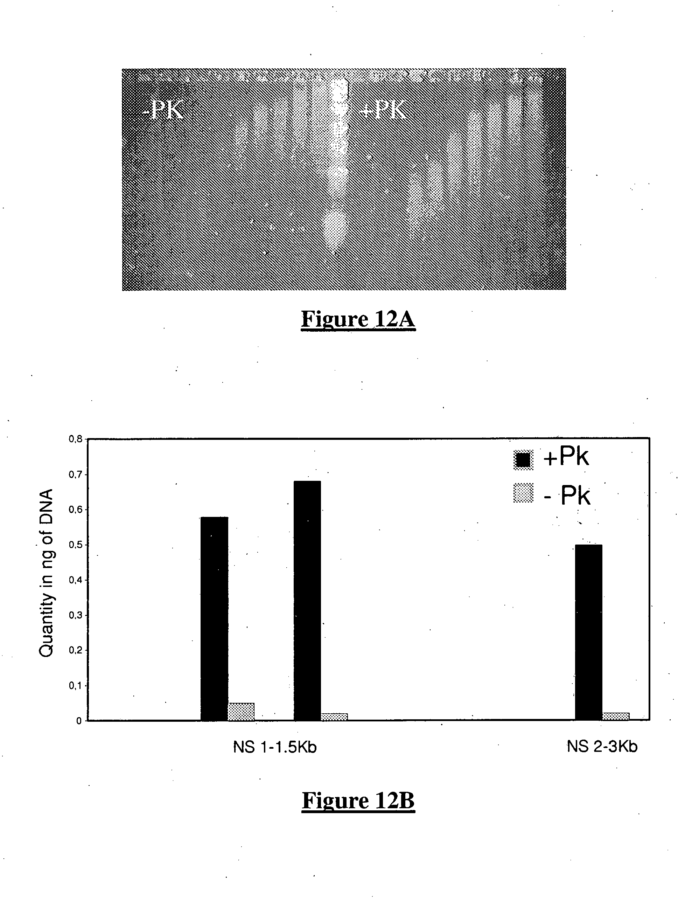

### Figures 12 A- F represent the improvement of purification steps of nascent strains

Figure 12A represents the migration in an agarose gel of nascent strands recovered at the end of the purification after sucrose fractionation, after treatment (+PK) or not (-PK) of cell lysate obtained with DNAzol, with T4 PNK kinase.
Figure 12B represents an histogram showing the increase of the amount and enrichment of nascent strands on hoxB9 locus. NS means Nascent strands. Black columns correspond to DNA treated with T4 PNK, and grey columns correspond to non treated DNA.

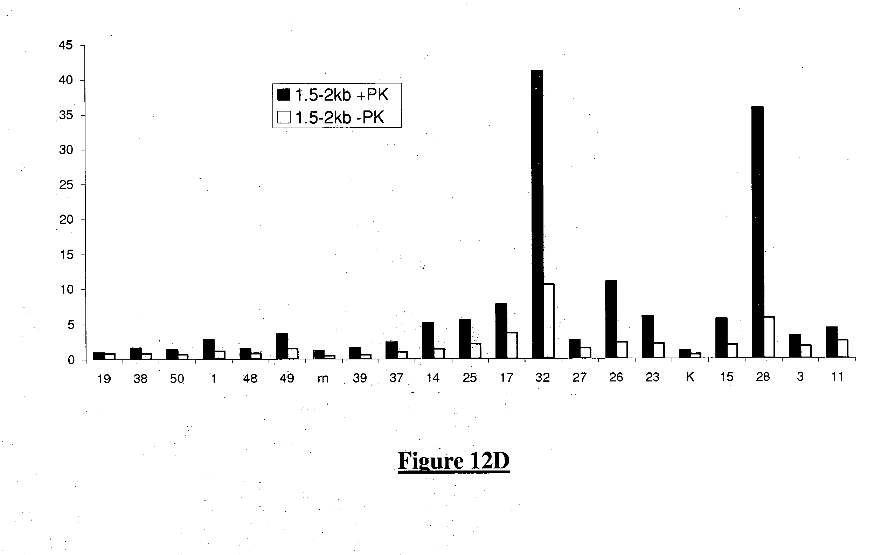

Figure 12D represents Hoxb9 locus. Black boxes represent genes and triangles represent primers used for qPCR. Scale: in kilobases
Figure 12D represents an histogram showing the increase of enrichment after second round of T4 PNK + lamda exonuclease treatment on hoxb9 origin.Y-axis corresponds to enrichment.

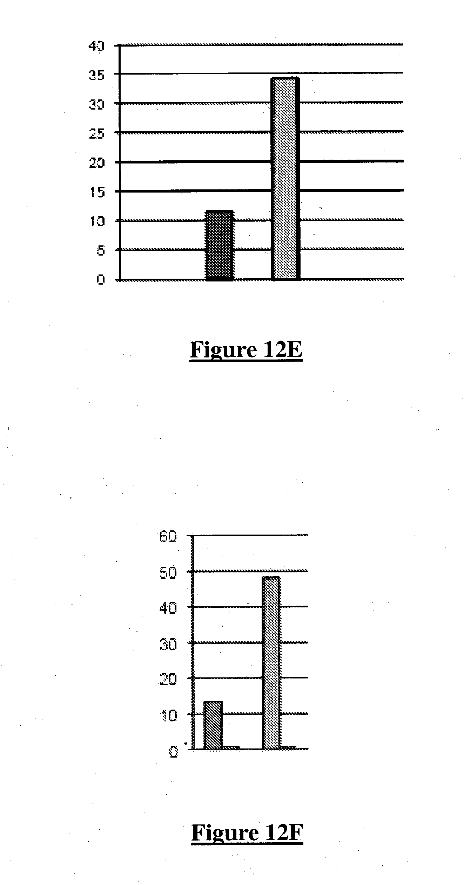

Figure 12E represents an histogram showing the increase of enrichment, of nascent strands of 1-1.Skb after second round ofT4 PNK + lamda exonuclease treatment on hoxb9 origin. Y-axis corresponds to enrichment. NS means nascent strand.
Figure 12F represents an histogram showing the increase of enrichment, of nascent strands of 1-1.Skb after second round of T4 PNK + lamda exonuclease treatment on e-mye origin. Y-axis corresponds to enrichment. NS means nascent strand.

### EXAMPLES

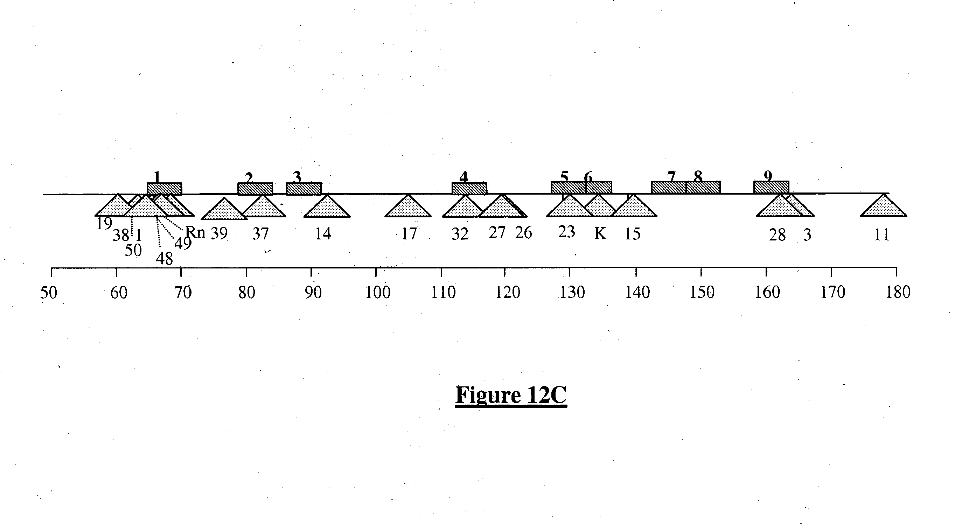

### Example 1: Protocole for nascent DNA purification (Figures 11 and 12)

### Precipitation DNA

- dividing cells (2.5 x 108 to 5 x 108 = 2* 150mm) were washed with PBS.
- cells were harvested and lysed in 15 ml of DNAzol® for 5 min at room Temperature (RT)
- Proteinase K was added in DNAzol to 200 µg/ml, and incubated at 37°C 2 hours. Centrifugation at 4000RPM, 15 min and the supernatant is rescued.
- To the supernatant, 15 ml of ethanol 100% were added to precipitated for S min at RT.
- Spooled out the DNA using a drawn pasteur pipette in a tube with 5ml of ethanol 70% for 5 min at RT
- spooled out the DNA using a drawn pasteur pipette in a new dry tube 2ml to dry the pellet (30 min at RT).

| | |
|---|---|
| DNA is resuspended in 2 ml of TEN20 at 70°C | tris 1 O mM pH7. 9 final |
| | EDTA 2mM final |
| | NaCl 20mM final |
| | SDS 0.1% |
| | RNasin 1000U |

The solution was boiled for 10-15 min, chilled on ice

### Sucrose gradiant NS purification

| | |
|---|---|
| Load 1mL onto a single neutral 5 to 30% sucrose gradient prepared in TEN500 in a 38.5-ml centrifuge tube. | tris 10 mM pH7.9 final |
| | EDTA 2mM final |
| | NaCl 300mM final |

Gradients were centrifuged in a Beckman SW28 rotor for 20 hat 24 000 rpm at 4°C. 1 ml Fractions were withdrawn from the top of the gradient using a wide-bore pipette tip 50 µl of each fraction was run with appropriate size markers on a 2% alkaline agarose gel, ON at 4°C at 40-50 volt.
neutralized gel with TBE1X and stained with GelRed.

Fractions corresponding to 0.5-1 kb, 1-1.5 kb, 1.5-2 kb and 2-3 kb were rescued and precipitated with 2.5 Vol of ethanol 100% 15 min at -80°C.
Pellets were washed with I ml of ethanol 70% and resuspended in 20 µl of water with 100U of RNasin.

### DNA contaminant withdrawn

**1**-After addition of2 µl Buffer PNK (New England Biolabs), fractions were boiled for 5 min, chilled on ice,

| | | |
|---|---|---|
| **2**-phosphorylation with T4 polynucleotide kinase in a volume of 1 OO µl final vol | | |
| T4 mix: | water | qsp 80ul |
| | Buffer PNK NEB 10X | 1X |
| | ATP | 50nM (0,05ul of 100mM) |
| | T4 PNK | 20U (2ul of 10U/ul) |

The reaction is incubated at 37°C for 1H,15 min at 75°C and directly precipitated by ethanol (2.5vol)-Na-acetate (0.3M) for 15min at -80°C.
**3**-Pellets were washed with 1 ml of ethanol 70% and resuspended in 50 µl of water with 100 U of RNasin.

| | | |
|---|---|---|
| **4**-The remainder is digested with 5 µl of lambda exonuclease in a final volume of 100 µl | | |
| Lambda exo mix : | water | qsp 50ul |
| | L-exo buffer 1 OX | 1X |
| | L-exo (Fermentas 20 U/µl) | 5ul |
| | BSA | IX (1ul of 100X) |

Fermentas L-exo buffer
67 mM glycine-KOH (pH 9.4)
2.5 mM MgC12
50 µg of bovine serum albumin per ml)

The reaction is incubated overnight at 37°C.

Aliquots of both the digested DNA and the undigested control were run on an 2% agarose gel.

5-the nascent strands were extracted once with phenol/chloroform/JAA and once with chloroform/JAA, and ethanol (2.5vol)-Na-acetate (0.3M) precipitated for 15min at -80°C.
6-Pellets were washed with 1 ml of ethanol 70% and resuspended in 20 µl of water.
7-The NS is subjected to another step of phosphorylation by T4 PNK and lamda-exo digestion (2- to 5-)
8-The final NS resuspended in 50 µl of tris 10mM is directly quantified with Roche-LC480.

### Example 2 : Nascent strands amplification (Figures 11 and 12)

Purification of Nascent Strand with Cyscibe-GFX kit
Elution in 50 ul
use 1 Oul and amplify with WGA-Sigma kit without the first fragmentation step.
Purify amplicons with nucleospin kit with a 1/5 dilution in NBA buffer prior to fix on column.
Elution in 50µ1.

LC480 (Light cycler 480) on 0.1 a 0.5ul of the amplicon.

### Example 3: Genome-wide analysis of replication origins in five different cell types reveals several choices but a conserved repeated element Introduction

In metazoans, thousands of chromosomal sites are activated at each cell cycle to initiate DNA synthesis and permit total duplication of the genome. They all should be activated only once to avoid any amplification and maintain genome integrity. How these sites are defined remains elusive despite considerable efforts trying to unravel a possible replication origin code. In *Saccharomyces cerevisiae*, DNA replication origins are specifically identified by specific DNA elements, called Autonomous Replication Sequence elements (ARS), which have a common AT-rich 11 bp specific consensus. However, sequence specificity identifies but not determines origin selection. Indeed, of the 12,000 ACS sites present in *S*. *cerevisiae* genome only 400 are functional [Nieduszynski CA, et al. Genes Dev. 2006 Jul 15;20(14):1874-9]. In S. pombe, ARS elements were also identified but they do not share a specific consensus sequence like in *S*. *cerevisiae.* Here, DNA replication origins are characterized by AT-rich islands [Dai J, et al. Proe Natl Acad Sci US A. 2005 Jan 11;102(2):337-42*;* Heichinger C, et al. EMBO J. 2006 Nov 1; 25(21): 5171-9] and poly-dA/dT tracks.
In multicellular organisms, it was more difficult to identify common features of DNA replication origins. No consensus sequence element has been found, which can have predictive value, although specific sites are recognized as DNA replication origins in chromosomes of somatic cells. It was soon suspected that metazoan ORis might be linked to other genetic features of complex organisms as the requirement to coordinate DNA replication not only with cell growth but also cell differentiation, and correlations with transcription and/or chromatin status have been found [Cayrou C, et al. Chromosome Res. 201 O Jan; 18 (1): 13 7-45]. However, identification of replication origins has been hampered by the lack of a genetic test as the ARS test in yeast, and methods to map replication origins which were not always adapted to a robust genome-wide analysis. First recent genome-wide studies to map origins in mouse and human cells (Cadoret et al., 2008; Sequeira-Mendes et al., 2009) have observed a correlation with unmethylated CpG islands regions as well as some overlap with promoter regions [Sequeira-Mendes J, et al. PLoS Genet. 2009 Apr; 5(4): e1000446]. However, it is not clear whether CpG islands are here a specific mark of replication origins or of the associated transcription promoters.

The Inventors tried to reveal new features of eukaryotic origins, first by upgrading the method used to map nascent stands DNA at origins to a specificity and reproducibility compatible with a genome-wide analysis compatible with the use of tiling arrays. Then, the Inventors used this method for four kinds of cell systems: mouse embryonic stem cells (ES), mouse teratocarcinoma cells (P19), mouse differentiated fibroblasts (MEFs), and Drosophila cells (Kc cells). The aim of using mouse cells and drosophila cells was to possibly detect conserved features in evolution and the aim of using mouse cells in different cell behaviours was to analyze the contribution to differentiation as opposed to pluripotent cells.

### Genome-wide replication origins maps

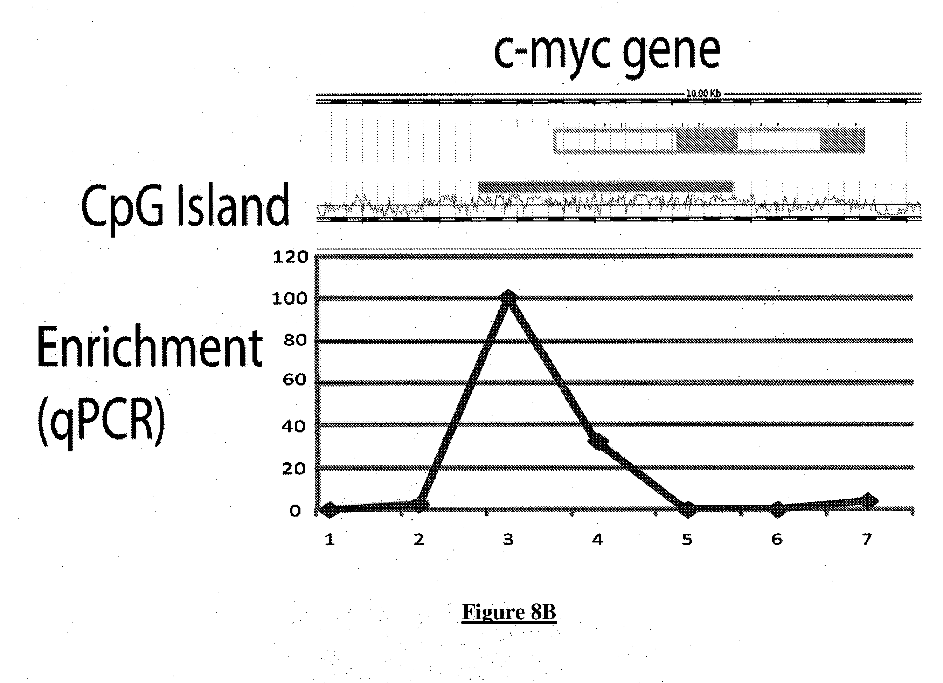

The RNA-primed nascent DNA procedure of preparation was initially improved using P 19 cells that grow in large amounts, and the method is detailed in Supplementary material and Figure 6A-E. It was checked with up to 5 entirely different duplicates.
Nascent strand preparations were hybridized on tiling micro-array (Nimblegen, oligonucleotides spaced every 100 bp). The full data set consists of continuous 60.4 Mbp on mouse chromosome 11 and 118.3 Mbp of Drosophila genome. Origins maps show enrichment at specific genomic locations with a high degree of reproducibility (Fig 1A-C and Fig7A-C). The Inventors validated the Ori maps of known origins by qPCR analysis of mouse e-Mye gene (Fig 8B) and HoxB domain (Fig 8A) as well as of randomly chosen putative Oris (Fig 8C-F). No specific signals was observed when total DNA or 'Nascent Strands' from mitotic cells was used for hybridization (Fig IB, IC and Fig 9), or when NS was RN Ase treated before exonuclease digestion (data not shown), confirming the specificity of our purification procedure. Importantly, no replication origin could be detected when using 'nascent strands' purified from non-cycling mitotic cells, confirming the specificity of our purification scheme. The Inventors identified 3299, 3263, 1896 and 8460 origins from ES, PI 9, MEF and Kc cells respectively

### Replication origins distribution

The method used allows scoring potentially all activated origins activated during the whole S-phase as exponentially growing cells were used. If there is existing variation between the origins activated in a given cell relative to another in the same growing cell population, all the potential replication initiation sites will be scored. In such conditions, the Inventors scored 146700 potential origins per genome, similar for the both mouse pluripotent cell types (Fig 1b, but MEF cells display significantly less origins, 84800 potential origins per genome (Fig Ib, and this is associated with an increase in origin length. 60.2% MEF origins were also observed in the two pluripotent cell lines cell lines.
Replication origins of Drosophila cells display the same length than MEF cells (4303 bp versus 4480 b) but with density higher than mouse cells (see later).
With regard to genes, mouse replications origins were found to be significantly associated with genes (p<0.001; Fig ID). More particularly, origins overlap significantly (p<0.001) promoter and exonic sequences in all murine cell types (Fig IE). Drosophila origins were found associated significantly with exonic sequences (Fig 1 G). Highly transcribed genes are enriched in replication origins, suggesting that transcription may facilitate origin specification and/or firing (Fig IH and 11).

### Replication origins are determinate by CpG island-like regions

Given their association with transcriptional units and with promoter regions, the Inventors examined the distribution of replication origins around the transcription start sites (TSS) in mouse cells. Overall, TSS are highly associated with nascent strands signals (Fig 2A, 10A and 10E). Strikingly, the Inventors observed a strong bimodal distribution around the TSS, with a low probability to get nascent strands overlapping the TSS, whereas the two borders were enriched. This suggests that, at these DNA replication origins, two nascent strands initiation sites are used, bordering the TSS. A possible explanation was that a genetic element at the TSS was not itself used as a DNA synthesis site but driving initiation on its borders. In Drosophila cells, TSS are not enriched in origins, in contrast to mouse cells (Fig 2E). In agreement, the Inventors did not observe the mouse bimodal distribution but detected an increase of origin density within gene as opposed to the promoter region (Fig 2E).
Mammalian promoters and particularly from highly expressed genes are CpG-rich while genes highly regulated during development are often CpG-poor or free. CpG-rich sequences are known as CpG Islands (CGI). To better understand the bimodal distribution, the Inventors divided our analysis on TSS CpG-positive (n=820) and TSS CpG-free (n=434) separately. Notably, nascent strands specific signals are strongly associated with CGI-positive promoter while CG I-negative promoters are devoid of such signals in all three mouse cell lines (Fig 2B, 10B and 10F). The Inventors next looked at origins distribution around CGI. The Inventors found that replication origins are strongly associated with CGI in all mouse cell lines (Fig 2C, 2D, 10D and 10H). Moreover, origins distribution was also found to be bimodal around CGI (Fig 2H, 10C and 10G).
CGI are usually defined as regions of 200 pb min in length with 60% of CG-richness and a ratio of CpG observed/CpG >0.6. Because cytosine methylation is almost inexistent in drosophila melanogaster, there is not a genome-wide bias toward eliminating CpG dinucleotides during evolution. The drosophila genome nevertheless contains region with identical properties as mammalian CGI. The Inventors delimitated these regions as CGI-like sequences. More of the half of CGI-like regions (54%) in drosophila cells and more than 70% of these sequences in mouse cells lines are associated with replication origin. These values drop to 32% and 43% for the randomized origins dataset. Moreover, the population of origins that is longer than average is even more associated with this sequence (82% in mice, Fig 2I and 2K). Altogether, the strong association of replication origins with CpG Island positive and highly transcribed genes may suggest that active genes are occupied by components from the pre-replicative complex.
The Inventors concluded that sequences related to CGI are determinant for localization of origins in mice as well as drosophila, regardless of their genomic position, e. g. not only in promoter region, consistent with presence of CGI-like sequences in exonic region from drosophila genome. These results also provide a novel possible function for CGI sequences conserved both in vertebrates and invertebrate species.
Nevertheless, CpG island rich sequences does not recognize the majority of replication origins (see Fig 2D, 10D and 10H). The Inventors conclude that replication origins might be specified by additional mechanisms, and the primary sequence was one possibility.

### The majority of metazoan replication origin shares a common motif

No consensus sequence is known to be associated with metazoan origins. Nevertheless, the Inventors hypothesized that such a sequence could potentially be identified in drosophila origins because of the compactness of the fly genome. As a first approach, fifteen 3kb length origins sequence were submitted to the MEME server (http://meme. sdsc.edu/meme4 4 O/intro.html) using default settings. A repetitive O-rich motif was found. When matched on the drosophila genome, this motif detected a large (>50%) proportion of replication origins. Several rounds of optimization gave rise to a repeated O-rich sequence that contained G every three nucleotides along the repeat (Fig 3A). Because of its unique ability to detect Oris (see below) and of its repetitive nature, this motif was dubbed ORE for Origin Repeated Element. When the Inventors looked for the occurrence of this motif genome-wide (using FIMO server; http://meme.sdsc.edu/meme/fimo-intro.html), the Inventors found that it had good predictive value as it was associated to more than two thirds of the origins (Fig 3B). In contrast, changing the nucleotide position in the motif results in poor origins prediction, indicating that the primary sequence, and not only QC-content, was essential (Fig 3B and 3C). Interestingly, the repeat number influenced Ori prediction: increasing the number of repeats in ORE significantly improved prediction, whereas decreasing the repeat number lowered it (Fig 3H). Cumulative origins signals associated with the motif again revealed a bimodal distribution, similarly to CGI-like domain, but the motif detects more origins than these domains (Fig 3D). Moreover, the Inventors observed that NS signal associated with ORE was asymmetric, being more enriched at the 3'. The Inventors further observed that the motif found in drosophila cells was efficient for detecting the majority of replication origins mapped in MEF, ES and P19 cells (Fig3E and 3F). Permuting the motif position again strongly reduces origin coverage by the motif, confirming that the primary sequence of the motif was important. Nascent Strands signals around ORE showed an asymmetric bimodal distribution, like in drosophila cells (Fig 3G). Finally the Inventors found that ORE was present in the majority of the previously characterized Oris (Fig 3I and 3J). The ORE has a significant predictive power. Indeed, also two thirds of all ORE occurrences mapped very close to replication origins in drosophila cells. In mammals, the ORE is less efficient, but nevertheless it is one order of magnitude more predictive than the 3.3% predictive value of the ACS element in budding yeast. Altogether, these results suggest that metazoan replication origins display a conserved element which might be involved either for origin specification and/or origin activation/firing.

### Hieratical organization of replication origins in metazoan

Genome-wide data permit to identify sites which can serve as DNA replication origins, but do not permit to have a view of origin usage along individual DNA molecules. Analysis at the single molecule level can be performed by DNA combing, where replicating DNA is labeled with pulses of modified nucleotide in vivo, and high molecular weight then stretched at a constant rate onto a slide. This method allows the precise determination of replication speed and inter-origin distances (Fig 4). MEFs, ES and P 19 cells replicate their DNA with a similar fork speed of 1.5 kb/min, similar to rates observed in human cells. Drosophila cells exhibit a nearly two-fold slower fork (0.8 kb/min), possibly due to the lower.
Sequential dual nucleotide labeling to determinate fork direction and bi-directional origins of replication was performed. The Inventors observed a near two-fold difference in inter-origin distances between mouse cells (139 kb) and drosophila cells (73 kb) (Fig 4A-D). The smaller inter-origin distance in Kc cells might be a consequence of the more compact drosophila genome. Of note, pluripotent or differentiated mouse cells have similar inter-origins spacing even though they differ in cell cycle profile and origin repertoire.

If all mapped origins were activated (firing efficiency of 100%) the resulting very short inter-origin distance distribution would be significantly different from the distribution observed by DNA combing (Fig 4E and 4F). As exemplified by MEF cells, the origin density of all potential origins was 4.3 fold higher than the density observed by DNA combing; indicating that 1 in every 4.3 origins on average is fired in individual DNA molecules (firing efficiency of ∼23%). Our results both in Drosophila and mouse cells are consistent with the findings that metazoan origins, like yeast Oris [Heichinger C, et al. EMBO J 2006 Nov 1;25(21):5171-9], are redundant and that only a small proportion of them is effectively used at each cell cycle. The Inventors next wanted to model genome-wide origin usage in MEF cells to recapitulate the origins firing pattern observed in single cell. The Inventors first tested the possibility that origins were fired purely stochastically (Fig 4G). Using a firing efficiency of 23%, the mean inter-origin distances of randomly fired Oris was identical to the value obtained by DNA combing. However the simulated inter-origin distance distribution was significantly different from the distribution obtained in combing experiments (Fig 4J). The purely stochastic distribution was characterized by populations of short and long inter-origin distances not observed in combing experiments (arrows in Fig 4H). The group of large inter-origin distances was in agreement with the random gap problem, with the consequence that too large replicons could not completely replicate and that a large number of gaps of unreplicated DNA would persist at the end of S phase [Hyrien O, et al.Bioessays. 2003 Feb, 25(2):116-25*;* Laskey RA. J Embryol Exp Morphol. 1985 Nov,•89 Suppl:285-96]. In the second model that the Inventors called the hierarchical stochastic model, groups of adjacent origins are functionally linked together into domains over a defined distance that defines the replicon, where activation of one origin silences the others (Fig 4H). Origins were thus grouped, taking into account their distribution along the genome, and one single Ori per domain was allowed to fire randomly. Strikingly, the simulated inter-origin distances were significantly similar to the combing data (Fig 4L). Importantly, the hierarchical stochastic model was also functional in ES and in Kc cells (Fig 4I, 4K and data not shown). This model requires optimization of the clustering parameters (the average size of the cluster). Nevertheless, the model is thus robust and can accommodate changes in origin density and firing efficiency. Overall, these data suggest that DNA replication origins are in large excess in metazoans and have a flexible use. Metazoan replicons appear constituted of groups of potential and flexible adjacent origins where activation of one origin suppresses the surrounding ones.

### Density of replication origins in chromosome 11

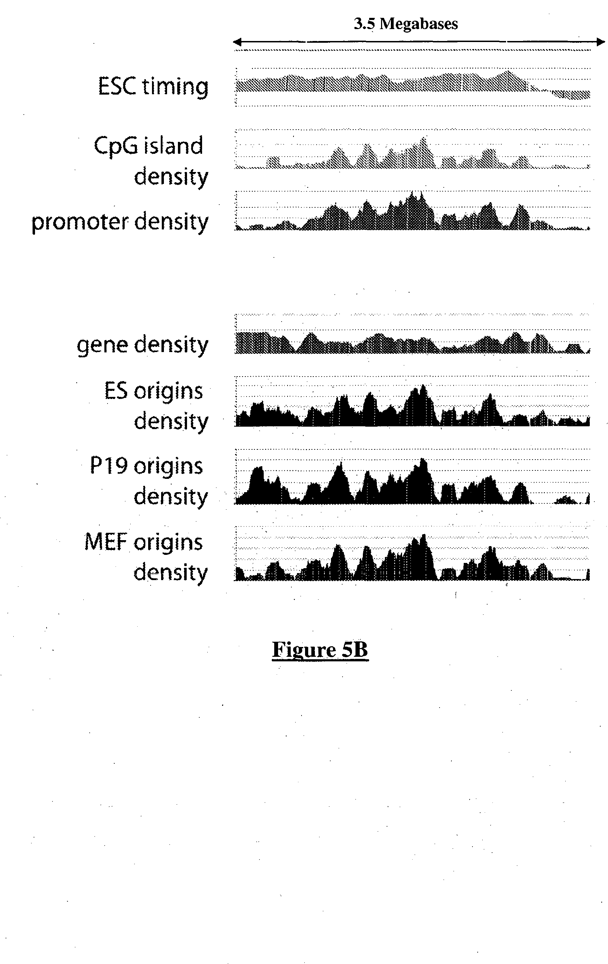

DNA replication origins are often synchronously activated in clusters. The Inventors looked at the origins density on areas of 70Kb in mice and 50Kb in Drosophila through a sliding window every 1 O bp. First, the Inventors observed that zones of high density of origins were at similar positions along chromosome 11, for all three mouse cells lines (Fig SA). Then, the Inventors compared these areas with other genomic features such as density in genes, promoter and CpG islands. For example, the areas of density origins coincide well with areas of density of CpG islands in MEF cells (Fig SA). A similar trend was observed for ES and P19 cells (data not shown). These data confirm that CpG islands are key to locate and/or fire replication origins. The replication timing of different ESC cells was recently published, and showed a very high conservation profile between distantly related pluripotent cells. The early regions were significantly associated with both a higher transcription level and a greater content in GC-rich sequences. The Inventors observed a strong correlation between early replicated regions and areas of high origins density in ES and Pl9 cells (Fig 5A). In MEFs, the Inventors also observed a strong correlation between early replicated regions and areas of high origins density (Fig 5A). For example, a 3.5 Mb early replicating domain is enriched in replication origins in all mouse cell lines tested (Fig 5B). This region is also rich in CpG Island, promoter and genes. In a late replicating part of chromosome 11, pluripotent cells display low density replication origins (Fig SC). However, MEP show strong origin activity, suggesting that this region could replicate early in this cell type. Similar, but albeit weaker, trends where observed for drosophila replication origins (data not shown).

The inventors thus propose that a replication cluster includes consecutive groups of adjacent flexible Oris, each set constituting a replicon, that are activated synchronously (see Fig 4H). The selection of a given Ori within each replicon might be achieved through several mechanisms. Selection itself might depend on the cell fate or the organization of the chromatin domain. The Ori interference mechanism has been described in yeast [Brewer and Fangman, Science. 1993 Dec 10;262(5140):1728-31*;* Lebofsky R, et al. Mol Biol Cell. 2006 Dec;17(12):5337-45] where firing at one Ori inhibits close-by Oris and this phenomenon could lead to the 100-120 Kbp average size of the replicon. Alternatively, control elements or chromosome organization might control firing in the cluster. For example, activation of one Ori might promote looping out of the replicon and silencing of the other potential Oris. The CpG Islands seems to be a putative control element for origin organization (Fig 5D).

## Claims

1. Use of an isolated nucleic acid sequence, as a multi cellular DNA replication origin wherein said nucleic acid sequence consists of
a) the nucleic acid sequence
5'- (N₇)ₐ(N₈)_{b}(N₃GN₄)_{c}(N₇)_{d}(N₈)ₑ-3' (SEQ ID NO: 2)
wherein N₃ is a T or a G base and N₄ is a G or a C,
wherein c vary from 3 to 20
wherein N₇ and N₈ represent any nucleotide,
wherein a and e independently from each other can be equal to 0, 1, 2 or 3, or vary from about 15 to 30, and
wherein b can be equal to 0, 1, 2 or 3 wherein d can be equal to 0, 1,2 or 3 or vary from about 10 to 300,
b) or any fragment of the above sequence consisting of at least 9 nucleotides, said nucleic acid sequence being such that
• it contains from 33% to 66% of G,
• it contains from 27% to 33% of C,
• it contains from 0% to 12% of A,
• it contains from 0% to 15% of T,
• it has a minimal consensus sequence 5'-N3GN4-3' wherein N3 is a T or a G base and N4 is a G or a C
said minimal consensus sequence being repeated from 3 to 20 times without interruption between said repeated minimal consensus sequence.

2. Use of the isolated nucleic acid sequence according to claim 1, wherein said nucleic acid sequence consists of one of the following sequences:
AGCTGGGGCGGCA (SEQ ID NO: 24)
CAGCTGGGGCGGC (SEQ ID NO: 25), and
GCTGGGGCGGCAG (SEQ ID NO: 26).

3. An isolated nucleic acid sequence as defined in claim 2, for its use for treating pathologies involving a deregulation of DNA replication.

4. The use of purified nascent DNA (hybrid RNA-DNA) for the implementation of a process allowing the mapping and the numbering of the active DNA replication origins of multi cellular eukaryotic cells, and the characterization of the sequence of said replication origins,
said process comprising
- a step of .extracting a mixture of nucleic acid molecules, said mixture of nucleic acid molecules comprising DNA and hybrid RNA-DNA, from multi cellular eukaryotic cells
- a step of enrichment of hydrid RNA-DNA from said mixture by eliminating proteins associated with said nucleic acid molecules, and
- at least two step of elimination of DNA from the mixture to recover purified nascent DNA,
wherein said nascent DNA are produced by the active replication origins, said replication origins being represented by the nucleic acid sequences as defined in claim 1 or 2.

5. A method for controlling in vitro the replication of a nucleotidic sequence into a pluricellular eukaryotic cell, including mammal cells, comprising the insertion of, into said nucleotidic sequence, a nucleic acid sequence as defined in claim 1 or 2.

6. The method according to claim 5, comprising a step of introducing said nucleotidic sequence into a pluricellular eukaryotic cell.

## Patentansprüche

1. Verwendung einer isolierten Nukleinsäuresequenz als einen multizellulären DNA-Replikationsursprung, wobei die Nukleinsäuresequenz besteht aus
a) der Nukleinsäuresequenz
5'-(N₇)ₐ(N₈)_{b}(N₃GN₄)_{c}(N₇)_{d}(N₈)ₑ-3' (SEQ ID NO: 2)
wobei N₃ eine T- oder eine G-Base ist und N₄ G oder C ist,
wobei c von 3 bis 20 variiert,
wobei N₇ und N₈ jedes beliebige Nukleotid repräsentieren,
wobei a und e unabhängig voneinander gleich 0, 1, 2 oder 3 sein können, oder von etwa 15 bis 30 variieren, und
wobei b gleich 0, 1, 2 oder 3 sein kann, wobei d gleich 0, 1, 2 oder 3 sein kann oder von etwa 10 bis 300 variiert,
b) oder jedem Fragment der obigen Sequenz bestehend aus wenigstens 9 Nukleotiden, wobei die Nukleinsäuresequenz so ist, dass
• sie von 33% bis 66% G enthält,
• sie von 27% bis 33% C enthält,
• sie von 0% bis 12% A enthält,
• sie von 0% bis 15% T enthält,
• sie eine minimale Konsensus-Sequenz 5'-N3GN4-3' hat, wobei N3 eine T-oder eine G-Base ist und N4 G oder C ist,
wobei die minimale Konsensus-Sequenz zwischen 3 bis 20 Mal ohne Unterbrechung zwischen der wiederholten minimalen Konsensus-Sequenz wiederholt ist.

2. Verwendung der isolierten Nukleinsäuresequenz gemäß Anspruch 1, wobei die Nukleinsäuresequenz aus einer der folgenden Sequenzen besteht:
AGCTGGGGCGGCA (SEQ ID NO: 24)
CAGCTGGGGCGGC (SEQ ID NO: 25), und
GCTGGGGCGGCAG (SEQ ID NO: 26).

3. Isolierte Nukleinsäuresequenz gemäß Anspruch 2 zur Verwendung beim Behandeln von Pathologien, die eine Deregulation von DNA-Replikation umfassen.

4. Verwendung gereinigter naszierender DNA (Hybrid-RNA-DNA) für die Implementierung eines Verfahrens, welches das Mappen und Nummerieren von aktiven DNA-Replikationsursprüngen von multizellulären eukaryotischen Zellen und die Charakterisierung der Sequenz dieser Replikationsursprünge ermöglicht, wobei der Prozess umfasst:
- einen Schritt des Extrahierens eines Gemisches von Nukleinsäuremolekülen, wobei das Gemisch von Nukleinsäuremolekülen DNA und Hybrid-RNA-DNA von multizellulären eukaryotischen Zellen umfasst,
- einen Schritt des Anreicherns von Hybrid-RNA-DNA aus dem Gemisch durch Entfernung von Proteinen, die mit den Nukleinsäuremolekülen assoziiert sind, und
- wenigstens zwei Schritte des Entfernens von DNA aus dem Gemisch, um gereinigte naszierende DNA zu erhalten,
wobei die naszierende DNA von den aktiven Replikationsursprüngen hergestellt wird, wobei die Replikationsursprünge durch die Nukleinsäuresequenzen wie in Anspruch 1 oder 2 definiert dargestellt werden.

5. Verfahren zum Kontrollieren der Replikation einer Nukleotidsequenz in einer plurizellulären eukaryotischen Zelle, einschließlich Säugerzellen, *in vitro*, umfassend das Einbringen einer Nukleinsäuresequenz wie in Anspruch 1 oder 2 definiert in die Nukleotidsequenz.

6. Verfahren gemäß Anspruch 5, umfassend einen Schritt des Einführens der Nukleotidsequenz in eine plurizelluläre eukaryotische Zelle.

## Revendications

1. Utilisation d'une séquence d'acide nucléique isolée, en tant qu'origine de réplication multicellulaire de l'ADN, où ladite séquence d'acide nucléique consistant en
a) la séquence d'acide nucléique
5'- (N₇)ₐ(N₈)_{b}(N₃GN₄)_{c}(N₇)_{d}(N₈)ₑ-3' (SEQ ID NO: 2) où N₃ est un T ou un G et N₄ est un G ou un C,
où c varie de 3 à 20
où N₇ et N₈ représentent n'importe quel nucléotide,
où a et e indépendamment l'un de l'autre peuvent être égale à 0, 1, 2 ou 3, or varier d'environ 15 à 30, et
où b peut être égale à 0, 1, 2 ou 3 où d peut être égale à 0, 1, 2 or 3 or varier d'environ 10 à 300,
b) ou n'importe quel fragment de la séquence ci-dessus consistant en au moins 9 nucléotides,
ladite séquence d'acide nucléique étant telle que
• elle contient de 33% à 66% de G,
• elle contient de 27% à 33% de C,
• elle contient de 0% à 12% de A,
• elle contient de 0% à 15% de T,
• elle a pour séquence consensus minimale 5'-N3GN4-3' où N3 est un T or un G et N4 est un G ou un C
ladite séquence consensus minimale étant répétée de 3 à 20 fois sans interruptions entre la ladite séquence consensus minimale répétée.

2. Utilisation d'une séquence d'acide nucléique isolée selon la revendication 1 où ladite séquence d'acide nucléique consiste en l'une des séquences suivantes:
AGCTGGGGCGGCA (SEQ ID NO: 24)
CAGCTGGGGCGGC (SEQ ID NO: 25), et
GCTGGGGCGGCAG (SEQ ID NO: 26).

3. Séquence d'acide nucléique isolée telle que définie dans la revendication 2, pour son utilisation pour traiter des maladies impliquées dans la dérégulation de la réplication de l'ADN.

4. Utilisation d'ADN naissant purifiés (hybrides ARN-ADN) pour la mise en oeuvre d'un procédé permettant la cartographie et le dénombrement des origines de réplication actives des cellules eucaryotes multicellulaires, et la caractérisation des séquences des origines de réplication,
ledit procédé comprenant
- Une étape d'extraction d'un mélange de molécules d'acide nucléique, ledit mélange de molécules d'acides nucléiques comprenant de l'ADN et des hybrides ARN-ADN, de cellules eucaryotiques multicellulaires,
- Une étape d'enrichissement des hybrides ARN-ADN dudit mélange en éliminant les protéines associées auxdites molécules d'acide nucléique, et
- Au moins deux étapes d'élimination de l'ADN du mélange pour obtenir de l'ADN naissant purifié
où lesdits ADN naissants sont produits par des origines de réplication actives, lesdites origines de réplication étant représentées par les séquences d'acide nucléique telles que définies dans la revendication 1 ou 2.

5. Méthode de contrôle de la réplication in vitro d'une séquence nucléotidique dans une cellule d'une eucaryote pluricellulaire, comprenant les cellules de mammifères, comprenant l'insertion, dans ladite séquence nucléotidique, d'une séquence d'acide nucléique telle que définie dans la revendication 1 ou 2.

6. Méthode selon la revendication 5, comprenant une étape d'introduction de ladite séquence nucléotidique dans une cellule d'eucaryote pluricellulaire.


### SEQUENCE LISTING

<110> CENTRE NATIONAL DE LA RECHERCHE SCIENTIFIQUE
<120> PURIFICATION PROCESS OF NASCENT DNA
<130> WOB 09 BJ CNR EUCA
<160> 31
<170> PatentIn version 3.5
<210> 1
   <211> 7
   <212> DNA
   <213> Artificial sequence
<220>
   <223> eukaryote replication origin
<220>
   <221> misc_feature
   <222> (1)..(2)
   <223> n is a, c, g, or t
<220>
   <221> misc_feature
   <222> (4)..(4)
   <223> N= G or A
<220>
   <221> misc_feature
   <222> (5)..(5)
   <223> N= T or C
<220>
   <221> misc_feature
   <222> (6)..(7)
   <223> n is a, c, g, or t
<400> 1
   nngnnnn 7
<210> 2
   <211> 7
   <212> DNA
   <213> Artificial sequence
<220>
   <223> eukaryote replication origin
<220>
   <221> misc_feature
   <222> (1)..(2)
   <223> n is a, c, g, or t
<220>
   <221> misc_feature
   <222> (3)..(3)
   <223> N= T or G
<220>
   <221> misc_feature
   <222> (5)..(5)
   <223> N= G or C
<220>
   <221> misc_feature
   <222> (6)..(7)
   <223> n is a, c, g, or t
<400> 2
   nnngnnn 7
<210> 3
   <211> 7
   <212> DNA
   <213> Artificial sequence
<220>
   <223> eukaryote replication origin
<220>
   <221> misc_feature
   <222> (1)..(2)
   <223> n is a, c, g, or t
<220>
   <221> misc_feature
   <222> (3)..(3)
   <223> N= G or C
<220>
   <221> misc_feature
   <222> (4)..(4)
   <223> N= T or A
<220>
   <221> misc_feature
   <222> (6)..(7)
   <223> n is a, c, g, or t
<400> 3
   nnnngnn 7
<210> 4
   <211> 21
   <212> DNA
   <213> Artificial sequence
<220>
   <223> eukaryote replication origin
<400> 4
   shgcygsygg mgcygshgst g 21
<210> 5
   <211> 21
   <212> DNA
   <213> Artificial sequence
<220>
   <223> eukaryote replication origin
<400> 5
   ckgykgckgc dgckgcdgyk g 21
<210> 6
   <211> 13
   <212> DNA
   <213> Artificial sequence
<220>
   <223> eukaryote replication origin
<400> 6
   gtcccagtcc cag 13
<210> 7
   <211> 13
   <212> DNA
   <213> Artificial sequence
<220>
   <223> eukaryote replication origin
<400> 7
   tgctgctgct gct 13
<210> 8
   <211> 13
   <212> DNA
   <213> Artificial sequence
<220>
   <223> eukaryote replication origin
<400> 8
   tatatatata tat 13
<210> 9
   <211> 13
   <212> DNA
   <213> Artificial sequence
<220>
   <223> eukaryote replication origin
<400> 9
   agcagcagca gca 13
<210> 10
   <211> 13
   <212> DNA
   <213> Artificial sequence
<220>
   <223> eukaryote replication origin
<400> 10
   gttgctgctg ctg 13
<210> 11
   <211> 13
   <212> DNA
   <213> Artificial sequence
<220>
   <223> eukaryote replication origin
<400> 11
   tcagacatct tag 13
<210> 12
   <211> 13
   <212> DNA
   <213> Artificial sequence
<220>
   <223> eukaryote replication origin
<400> 12
   agcagcagca aca 13
<210> 13
   <211> 13
   <212> DNA
   <213> Artificial sequence
<220>
   <223> eukaryote replication origin
<400> 13
   cagacatctt agg 13
<210> 14
   <211> 13
   <212> DNA
   <213> Artificial sequence
<220>
   <223> eukaryote replication origin
<400> 14
   agacatctta ggc 13
<210> 15
   <211> 13
   <212> DNA
   <213> Artificial sequence
<220>
   <223> eukaryote replication origin
<400> 15
   cagcagcagc agc 13
<210> 16
   <211> 13
   <212> DNA
   <213> Artificial sequence
<220>
   <223> eukaryote replication origin
<400> 16
   taacgtgtgg tga 13
<210> 17
   <211> 13
   <212> DNA
   <213> Artificial sequence
<220>
   <223> eukaryote replication origin
<400> 17
   tgttgctgct gct 13
<210> 18
   <211> 13
   <212> DNA
   <213> Artificial sequence
<220>
   <223> eukaryote replication origin
<400> 18
   cagcagcagc aac 13
<210> 19
   <211> 9
   <212> DNA
   <213> Artificial sequence
<220>
   <223> eukaryote replication origin
<400> 19
   tgctgctgc 9
<210> 20
   <211> 9
   <212> DNA
   <213> Artificial sequence
<220>
   <223> eukaryote replication origin
<400> 20
   cagcagcag 9
<210> 21
   <211> 9
   <212> DNA
   <213> Artificial sequence
<220>
   <223> eukaryote replication origin
<400> 21
   ctgctgctg 9
<210> 22
   <211> 12
   <212> DNA
   <213> Artificial sequence
<220>
   <223> eukaryote replication origin
<400> 22
   ctctctctct ct 12
<210> 23
   <211> 12
   <212> DNA
   <213> Artificial sequence
<220>
   <223> eukaryote replication origin
<400> 23
   tctctctctc tc 12
<210> 24
   <211> 13
   <212> DNA
   <213> Artificial sequence
<220>
   <223> eukaryote replication origin
<400> 24
   agctggggcg gca 13
<210> 25
   <211> 13
   <212> DNA
   <213> Artificial sequence
<220>
   <223> eukaryote replication origin
<400> 25
   cagctggggc ggc 13
<210> 26
   <211> 13
   <212> DNA
   <213> Artificial sequence
<220>
   <223> eukaryote replication origin
<400> 26
   gctggggcgg cag 13
<210> 27
   <211> 13
   <212> DNA
   <213> Artificial sequence
<220>
   <223> eukaryote replication origin
<400> 27
   agcagctgga cac 13
<210> 28
   <211> 13
   <212> DNA
   <213> Artificial sequence
<220>
   <223> eukaryote replication origin
<400> 28
   cagcagctgg aca 13
<210> 29
   <211> 13
   <212> DNA
   <213> Artificial sequence
<220>
   <223> eukaryote replication origin
<400> 29
   gcagcagctg gac 13
<210> 30
   <211> 13
   <212> DNA
   <213> Artificial sequence
<220>
   <223> eukaryote replication origin
<400> 30
   cagctggaca cac 13
<210> 31
   <211> 13
   <212> DNA
   <213> Artificial sequence
<220>
   <223> eukaryote replication origin
<400> 31
   agcagactgg gcg 13